# EUROPÄISCHE PATENTSCHRIFT

(11) **EP 3 553 161 B1**
(45) Veröffentlichungstag und Bekanntmachung des Hinweises auf die Patenterteilung: **07.07.2021**
(21) Anmeldenummer: 19167880.4
(22) Anmeldetag: 08.04.2019
(51) Int. Cl.: C12M 1/00, C12M 3/00

(54) **PHOTOBIOREAKTOR MIT VORRICHTUNG ZUR ABGABE ELEKTROMAGNETISCHER STRAHLUNG, VORRICHTUNG ZUR ABGABE ELEKTROMAGNETISCHER STRAHLUNG SOWIE VERFAHREN ZUR VERMEHRUNG ODER KULTIVIERUNG BIOLOGISCHEN MATERIALS, VERFAHREN ZUM PRÄPARIEREN VON BIOLOGISCHEM MATERIAL UND/ODER HERSTELLEN VON PHARMAZEUTIKA, INSBESONDERE BIOPHARMAZEUTIKA**
PHOTOBIOREACTOR WITH DEVICE FOR EMITTING ELECTROMAGNETIC RADIATION, DEVICE FOR EMITTING ELECTROMAGNETIC RADIATION AND METHOD FOR PREPARING OR CULTIVATING BIOLOGICAL MATERIAL, METHOD FOR PREPARING BIOLOGICAL MATERIAL AND/OR PRODUCING PHARMACEUTICALS, PARTICULARLY BIOPHARMACEUTICALS
PHOTOBIORÉACTEUR POURVU DE DISPOSITIF DESTINÉ À LA SORTIE DE RAYONNEMENT ÉLECTROMAGNÉTIQUE, DISPOSITIF DE SORTIE DE RAYONNEMENT ÉLECTROMAGNÉTIQUE AINSI QUE PROCÉDÉ DE REPRODUCTION OU DE CULTURE DE MATIÈRE BIOLOGIQUE, PROCÉDÉ DE PRÉPARATION DE MATIÈRE BIOLOGIQUE ET / OU DE PRODUCTION DE PRODUITS PHARMACEUTIQUES, EN PARTICULIER DE PRODUITS BIOPHARMACEUTIQUES

(30) Priorität: 09.04.2018 DE 102018108327
(43) Veröffentlichungstag der Anmeldung: 16.10.2019
(73) Patentinhaber: SCHOTT AG, 55122 Mainz (DE)
(72) Erfinder: OTT, Christian, 84539 Ampfing (DE); HETTLER, Robert, 84036 Kumhausen (DE)
(74) Vertreter: Blumbach · Zinngrebe Patentanwälte PartG mbB

(56) Entgegenhaltungen:
- WO-A1-2008/145719
- DE-U1- 8 703 478
- DE-U1-202008 005 412
- DE-U1-202009 010 255
- GB-A- 2 469 085
- US-A1- 2005 135 104

## Beschreibung

Die Erfindung betrifft einen Photobioreaktor mit einer Vorrichtung zur Abgabe elektromagnetischer Strahlung sowie eine Vorrichtung zur Abgabe elektromagnetischer Strahlung für einen Photobioreaktor.

Verfahren zur Herstellung biologischen Materials, wie beispielsweise biotechnologische Produktionsprozesse, welche die Kultivierung von Mikroorganismen, tierischen und pflanzlichen Zellen umfassen sind von zunehmender Bedeutung. Dies betrifft insbesondere die Herstellung von Biopharmazeutika oder auch von Functional Food, Designer Food oder Probiotica sowie Diagnostika, welche z. B. fluoreszierende oder (gelabelt) magnetische Signale induzieren können.

Mikroalgen werden beispielsweise im Produktionsmaßstab in Tanks oder Reaktoren, insbesondere in Röhrenreaktoren, kultiviert. Produktabhängig wird herkömmlich Sonnen- oder LED-Licht genutzt. LED-Licht ist erforderlich, wenn das Lichtspektrum der Sonne sich nicht für eine hinreichende Produktausbeute eignet. Im Allgemeinen steigen mit dem Wert eines Produktes auch die Anforderungen an dessen Produktionsbedingungen. Beispiele aus dem Bereich des Funktional Food sind die Herstellung von Fucoxanthin oder Astraxanthin oder auch von beispielsweise Phycoliproteinen für diagnostische Zwecke. Hierfür ist jedoch eine definierte Wellenlängenauswahl erforderlich. Für Astraxanthin wird die Wachstumsphase bzw. die Produktbildungsphase im Spektralbereich von blauem bis rotem Licht stimuliert.

Nach dem Stand der Technik ist die Nutzung von Mikroalgen für die Herstellung von Biopharmazeutika nicht möglich, da eine Kultivierung im Produktionsmaßstab obligat sterilen Produktionsbedingungen erfordert.

Das Dokument DE 10 2005 012 515 B4 betrifft eine Beleuchtungseinrichtung für Bioreaktoren, insbesondere für Inkubatoren zur Kultivierung von phototrophen Zellkulturen, welche außerhalb eines Bioreaktors angeordnet ist. Die Beleuchtungseinrichtung besteht im Wesentlichen aus einer Mehrzahl von ansteuerbaren Lichtquellen, die Licht unterschiedlicher Spektralbereiche abstrahlen. Dieses Dokument offenbart weiterhin ein Verfahren zur variablen Beleuchtung für die Kultivierung von phototrophen Zellkulturen in Bioreaktoren, insbesondere in Inkubatoren, mit einer eine Mehrzahl von Licht unterschiedlicher Spektralbereiche abstrahlenden Lichtquellen aufweisenden Beleuchtungseinrichtung.

Aus der DE 44 16 069 C2 ist ein Verfahren und ist eine Vorrichtung zum Ausleuchten von Medien bekannt, die zur Kultivierung von phototrophen Mikroorganismen und der Durchführung von photochemischen Prozessen mittels Licht in Behälter eingeführt sind, Die Vorrichtung umfasst seitlich abstrahlenden Lichtwellenleiter mit glatter Oberfläche, die zum Ausleuchten auf einer Trägervorrichtung angeordnet sind und die das Licht auf das Medium abstrahlen, damit eine Umwandlung des Mediums im Behälter erreicht wird. Die Vorrichtung zur Ausleuchtung, der Behälter und das Medium im Behälter können gemeinsam thermisch sterilisiert und wieder auf Kultivierungstemperatur abgekühlt werden, wobei die Lichtwellenleiter in einem thermischen Arbeitsbereich zwischen -20°C und +200°C wirksam bleiben. Das in die Lichtwellenleiter eingekoppelte Licht stammt von einer außerhalb des Behälters angeordneten Lichtquelle.

Das Dokument US 2005/135104 offenbart einen Photobioreaktor mit einer LED-Anordnung auf einem Träger in einem Gehäuse, welches transparent und wasserdicht ausgebildet ist.

Die Patentschrift DE 44 23 302 C1 offenbart eine Vorrichtung zur Einkopplung von Strahlungsenergie einer Lichtquelle in einen Photoreaktor, welcher ein Rohr aus lichtdurchlässigem Material, z. B. Glas umfasst. Zur Durchführung photochemischer Synthesen wird eine fokussierende holographische Vorrichtung verwendet, die mindestens einen wellenlängenselektiven Anteil der Strahlungsenergie auf das Innere des Reaktors bündelt.

Das deutsche Patent DE 10 2010 014 712 B3 offenbart einen modularen Photo-Röhrenreaktor zur photochemischen Behandlung fluider Medien, der eine zentralaxiale Bestrahlungseinheit umfasst, die zumindest eine Strahlungsquelle aufweist. Die Bestrahlungseinheit ist von einer aus Glas bestehenden Reaktorwand koaxial umgeben und weist zwischen der Reaktorwand und der Bestrahlungseinheit einen Ringspalt auf, der ein Bestrahlungsvolumen bereitstellt. Auch diese Bestrahlungseinheit ist außerhalb des Photo-Röhrenreaktors angeordnet, denn dieser umgibt diese zwar, ist aber strömungsmechanisch nicht in die Fluiddynamik Röhrenreaktors eingebunden.

Der Erfindung liegt die Aufgabe zugrunde, einen Photobioreaktor mit einer Vorrichtung zur Abgabe elektromagnetischer Strahlung sowie eine Vorrichtung zur Abgabe elektromagnetischer Strahlung für einen Photobioreaktor bereitzustellen, welche eine möglichst effiziente Nutzung des Photobioreaktors ermöglichen und bei welchen eine Kontamination des Inneren des Photobioreaktors vermindert wird.

Diese Aufgabe wird jeweils mit dem in den unabhängigen Ansprüchen beschriebenen Gegenstand gelöst. Vorteilhafte Weiterbildungen sind den abhängigen Ansprüchen sowie der weiteren Beschreibung zu entnehmen.

Mit einem Photobioreaktor, der einen Behälter zur Aufnahme von fluiden Medien, die biologisches Material enthalten, eine Vorrichtung zur Abgabe elektromagnetischer Strahlung, mit einem Gehäuse, in welchem eine Quelle zur Abgabe elektromagnetischer Strahlung angeordnet ist und ein oder mehrere Fenster aufweist, welche jeweils eine Einglasung ausbilden, umfasst und bei welchem die Vorrichtung zur Abgabe elektromagnetischer Strahlung mit deren Gehäuse innerhalb des Behälters zur Aufnahme von biologischem Material angeordnet ist, kann nicht nur elektromagnetische Strahlung in den Photobioreaktor eingeleitet, sondern darüber hinaus auch Einfluss auf die Fluiddynamik der in dem Reaktor angeordneten Medien Einfluss genommen werden.

Bei einem Photobioreaktor mit Durchführungen, insbesondere zumindest einer Durchführung, welche durch zumindest eine Wand des Photobioreaktors führt und bei welchem die Vorrichtung zur Abgabe elektromagnetischer Strahlung an zumindest einer Durchführung gehalten ist, können Zuleitungen und/oder Kontrollleitungen für die Quelle der elektromagnetischen Strahlung aus dem Äußeren des Photobioreaktors in diesen hinein und aus diesem heraus geführt werden. Besonders vorteilhaft können diese Durchführungen als Standard-Sparger-Ports ausgebildet sein, wodurch auch eine einfachere Nachrüstung herkömmlicher Photobioreaktoren mit der erfindungsgemäßen Vorrichtung zur Abgabe elektromagnetischer Strahlung unterstützt wird.

Um den strengen Anforderungen für die Herstellung von Biopharmazeutika zu genügen, kann das Material, welches der Photobioreaktor und welches die Vorrichtung zur Abgabe elektromagnetischer Strahlung jeweils umfassen so ausgewählt sein, dass dieses applikationsabhängig jeweils einem oder mehreren der nachfolgenden Standards entspricht:
i) FDA approved materials (ICH Q7A, CFR 211.65(a) - Code of Federal Regulations, USP Class, animal derivative free, bisphenol A free)
ii) EMA (European Medicines Agency) EU GMP Guide Part II approved materials
iii) Sectoral chemical resistance - ASTM D 543-06
iv) Biocompatibility e.g. referred to US Pharmacopeia or tests referred to ISO 10993.

Vorteilhaft ist es dabei, wenn der Behälter zur Aufnahme von fluiden Medien Edelstahl umfasst oder aus Edelstahl besteht. Verwendbar sind alle Edelstähle, insbesondere auch austenitische und ferritische Edelstähle, bevorzugt jedoch nur soweit diese bei der Durchführung der Erfindung rostfrei bleiben.

Ferner sind prinzipiell auch Titan sowie Monell-Legierung mit hohem Kupferanteil verwendbar, wobei das Material bei Verwendung für die Vorrichtung zur Abgabe elektromagnetischer Strahlung, insbesondere für deren Gehäusekörper auch emailliert sein kann.

Auch der Photobioreaktor kann emailliert sein, insbesondere auf der Innenseite des Behälters zur Aufnahme von biologischem Material.

Bevorzugt weisen die Oberflächen, welche mit den das biologische Material enthaltendem Fluid in Kontakt treten, Rauhigkeitswert von 0,8 oder geringer auf.

Wenn die Vorrichtungen zur Abgabe elektromagnetischer Strahlung mit deren Gehäuse beabstandet zu einer Wand des Behälters zur Aufnahme von biologischem Material angeordnet sind, können beispielsweis Prallbleche ersetz werden und kann auf diese Weise besonders vorteilhaft fluiddynamisch Einfluss auf die Strömung des fluiden Mediums genommen werden.

Vorteilhaft ist es hierbei, wenn der Photobioreaktor in seinem Inneren, insbesondere dem Inneren des Behälters zur Aufnahme biologischen Materials zumindest eine Montageeinrichtung zum Fixieren, insbesondere von Prall-oder Verwirbelungsblechen aufweist und die zumindest eine Vorrichtung zur Abgabe elektromagnetischer Strahlung mit zumindest dieser einen Montageeinrichtung verbunden ist.

Wenn der Photobioreaktor zusammen mit der Vorrichtung zur Abgabe elektromagnetischer Strahlung autoklavierbar ist, insbesondere während diese mit deren Gehäuse innerhalb des Behälters zur Aufnahme von biologischem Material angeordnet ist, autoklavierbar ist, kann hierdurch eine Kontamination mit biologisch aktivem oder wechselwirkendem Material mit sehr hoher Sicherheit ausgeschlossen werden.

Hierzu ist insbesondere auch die Vorrichtung zur Abgabe elektromagnetischer Strahlung autoklavierbar ausgebildet. Überraschend hat es sich gezeigt, dass bei den hier offenbarten Sensoraufnahmen zum Halten von zumindest einem Sensor 3500 Äuktoklavierungs-Zyklen bei 2 bar und 134 °C möglich waren.

Autoklavierbar wird im Rahmen dieser Offenbarung auch als autoklavierbar im Sinne der DIN EN ISO 14937; EN ISO 17665, welche für Medizinprodukte gültig ist, verstanden.

Bei einer besonders bevorzugten Ausführungsform sind hierzu innerhalb des Behälters zur Aufnahme von biologischem Material mehrere Vorrichtungen zur Abgabe elektromagnetischer Strahlung, vorzugsweise symmetrisch zur Längsachse des Behälters angeordnet. Hierbei kann auch eine homogenere Ausleuchtung des fluiden Mediums und das in diesem befindliche biologische Material erreicht werden.

Bezüglich der erfindungsgemäßen Vorrichtung zur Abgabe elektromagnetischer Strahlung für einen Photobioreaktor ist ein Gehäuse umfasst, in welchem die Quelle zur Abgabe elektromagnetischer Strahlung angeordnet ist. Hierdurch kann die Vorrichtung zur Abgabe elektromagnetischer Strahlung kontaminationsfreie Kultivierungs-Bedingungen innerhalb des Photobioreaktors ermöglichen.

Vorteilhaft umfasst die Quelle zur Abgabe elektromagnetischer Strahlung LEDs, insbesondere einen Array von LEDs, mit welchem ein vergrößertes Volumen innerhalb des Photobioreaktors definiert ausgeleuchtet werden kann. Die LED-Arrays liefern hierbei die für die optimierte Kultivierung von Mikroalgen erforderliche Lichtenergie in das Innere Edelstahl-Bioreaktors. Ferner können die LED-Arrays bedarfsorientiert in verschieden Wellenlängen emittieren und dadurch den Prozess der Wachstums- und Produktbildungsphase gezielt steuern.

Ebenso vorteilhaft umfasst die Vorrichtung zur Abgabe elektromagnetischer Strahlung zumindest einen Sensor zum Messen der Wellenlänge und/oder Intensität von elektromagnetischer Strahlung und bevorzugt ist der zumindest eine Sensor zum Messen von elektromagnetischer Strahlung in dem Gehäuse angeordnet, wobei mittels des Sensors im Betriebszuatand bevorzugt die Intensität und/oder Wellenlänge von einfallender elektromagnetischer Strahlung messbar ist. Mittels der vom Sensor erhaltenen Signale ist die Intensität der einfallenden elektromagnetischen Strahlung einstellbar, insbesondere geregelt, vorzugsweise mittels einer Rückkoppelungsanordnung geregelt einstellbar. Die Signale des Sensors können durch die Durchführung geleitet werden, insbesondere in Form von Kontrollleitungen. Der Sensor kann genutzt werden, um einerseits die Intensität und/oder Wellenlänge der von den LEDs emittierten Strahlung zu messen. Da diese einem Alterungsprozess unterliegen und/oder deren Emissionsspektrum z.B. von der Umgebungstemperatur abhängen kann, ist deren Emission im Betriebszustand mit Hilfe des Sensors steuerbar. Der zumindest eine Sensor kann aber auch dazu genutzt werden, um den Zustand des biologischen Materials im Bioreaktor zu kontrollieren und/oder zu charakterisieren. Durch Rückstreuung des im Betriebszustand beleuchteten biologischen Materials kann beispielsweise die Wellenlänge der vom Sensor empfangenen elektromagnetischen Strahlung Informationen über das biologische Material liefern. Wird das biologische Material, beispielsweise bestimmte Algen beispielsweise mit weißem Licht für insbes. deren Wachstum beleuchtet, streuen diese beispielsweise grünes Licht zurück. Die Intensität des grünen Lichts kann von der Konzentration der Algen und/oder deren Wachstumszustand abhängig sein. Diese Informationen können genutzt werden, um die Prozesse im Bioreaktor zu steuern, beispielsweise Nährstoffzufuhr, Temperatur usw.. Ebenso möglich ist selbstverständlich auch das Messen von Fluoreszenz usw.. Um die von den Strahlungsquellen im Betriebszustand emittierte Strahlung mit Sensorsignalen korrelieren zu können sieht es eine erfindungsgemäße Vorrichtung ebenso vor, dass ein Anteil der von den Strahlungsquellen im Betriebszustand emittierten Strahlung auf einen beschriebenen Sensor geleitet werden, beispielsweise durch Strahlteiler und/oder Lichtleiter. Die Mittel zum Leiten der emittierten Strahlung auf den Sensor können in dem Gehäuse integriert sein.

Ferner kann die Vorrichtung zur Abgabe elektromagnetischer Strahlung auch über einen oder mehrere Sensoren zur Messung des CO₂-Gehaltes innerhalb des Bioreaktors verfügen.

Ebenso vorteilhaft ist es, wenn der Bioreaktor mit mehr als einer Vorrichtung zur Abgabe elektromagnetischer Strahlung bestückt ist. Dies kann eine verbesserte Ausleuchtung des Innern des Photobioreaktors bewirken. Insbesondere ist es dann auch möglich, dass die Sensoren der Vorrichtungen zur Abgabe elektromagnetischer Strahlung nicht nur die Rückstreuung des im Bioreaktors befindlichen Materials messen können, sondern auch in Transmission. Ein Sensor einer Vorrichtung zur Abgabe elektromagnetischer Strahlung misst dann sozusagen die von der anderen Vorrichtung zur Abgabe elektromagnetischer Strahlung emittierte und von dem in dem Bioreaktor befindlichen Material beeinflusste, insbesondere transmittierte und/oder gestreute Strahlung.

Durch die Auswertung der Beleuchtungs- und/oder Sensorsignale über die Zeit lassen sich die in dem Photobioreaktor stattfindenden Prozesse mit erhöhter Genauigkeit und insbesondere abhängig von den Ergebnissen der stattfindenden Reaktionen des sich darin befindlichen biologischen Materials steuern. Dies kann zu einer erheblichen Ausbeuteverbesserung beitragen. Generell ist eine vorteilhafte Regulierung der reellen Intensität im Fluid, sogar ortsaufgelöst möglich, insbesondere ist dies auch mit einer dynamischen Anpassung an die Sollintensität durchführbar. Es kann ferner eine Kompensation der variierenden Ausleuchtungstiefe, welche durch die wechselnde/steigende Zelldichte während der Kultivierung bedingt ist, vorgenommen werden. Ebenso ist eine Kompensation durch Substratverbrauch und Produktbildung, welche eine bedingte Adsorptionsänderungen im Fluid bewirken kann, möglich.

Wenn die LEDs und/oder der zumindest ein Sensor hinter einem oder mehreren Fenstern des Gehäuses angeordnet sind, kann das Gehäuse der Vorrichtung zur Abgabe elektromagnetischer Strahlung mechanisch stabil und thermisch belastbar ausgeführt werden, sodass sowohl eine Autoklavierung als auch eine Reinigung und Sterilisierung mittels der als CIP (Clean-In-Place) und als SIP (Sterilization-In-Place) bezeichneten Verfahren ermöglicht wird.

Für die Fluiddichtigkeit und insbesondere die Hermetizität der Vorrichtung zur Abgabe elektromagnetischer Strahlung ist es von großem Vorteil, wenn ein oder mehrere Fenster jeweils eine GTMS-Druckeinglasung (Glass-to-Metal-Seal-Druckeinglasung) ausbilden. Hierdurch wird sowohl die mechanische Stabilität, die chemische Beständigkeit als auch die thermische Belastbarkeit erheblich gefördert.

Bei einer bevorzugten Ausführungsform ist zumindest ein transparentes Element eines Fensters scheibenförmig ausgebildet und weist insbesondere planparallele Hauptoberflächen auf.

Wenn jedoch das transparente Element plankonvex, plankonkav, bikonvex, bikonkav, konvexkonkav oder konkavkonvex geformt ist, kann hierdurch das Strahlungsfeld der elektromagnetischen Strahlung definiert in dessen räumlicher Verteilung beeinflusst und eine nochmals homogenere Ausleuchtung des Behälters des Photobioreaktors erreicht werden.

Bevorzugt umfasst das transparente Element des jeweiligen Fensters Glas oder besteht aus Glas. Hierbei kann das Glas des transparenten Elements des Fensters Quarzglas, Borosilicatglas oder Glassysteme umfassen oder aus diesen bestehen, welche eine hohe chemische Resistenz - vorrangig gegen Wasser- mit einer guten optischen Transparenz im gewünschten und nachfolgend angegebenen Wellenlängenbereich aufweisen.

Das transparente Element zumindest eines Fensters kann in einem Spektralbereich von 250 bis 2000 nm eine Transmission aufweisen, die höher ist als 80%, besonders bevorzugt höher als 90 %.

Eine fertigungstechnisch günstige sowie präzise Herstellung wird ermöglicht, wenn das Gehäuse einen Gehäusekörper umfasst, der als Frästeil ausgebildet ist.

Bevorzugt ist ein Grundkörper der Fenster direkt, insbesondere mittels Laser-Schweißen, jeweils hermetisch dicht mit dem Gehäusekörper verbunden und ist das Gehäuse hermetisch dicht ausgebildet. Hierdurch kann den für den Pharmabereich obligaten Materialanforderungen vorteilhaft Rechnung getragen werden.

Für ein möglichst großes ausgeleuchtetes Volumen des Behälters kann der Gehäusekörper säulenförmig ausgebildet sein, vorteilhaft mit dreieckigem Querschnitt, und es können zwei von dessen sich in Längsrichtung erstreckenden Seitenwänden einen 30° Winkel und zwei von dessen sich in Längsrichtung erstreckenden Seitenwänden einen 60° Winkel zueinander aufweisen. Vorteilhaft weist ein Bioreaktor zur bestmöglichen Ausleuchtung vier Vorrichtungen zur Abgabe von elektromagnetischer Strahlung mit solchen Gehäusekörpern auf.

Hierbei können zumindest zwei der sich in Längsrichtung erstreckenden Seitenwände Fenster umfassen.

Für einen modularen Aufbau kann das Gehäuse der Vorrichtung zur Abgabe elektromagnetischer Strahlung zumindest einen elektrischen Verbinder umfassen, welcher vorzugsweise anreihbar mit einem Verbinder eines weiteren Gehäuses ausgebildet ist. Um die Autoklavierung der Module zu gewährleisten werden auch die Verbinder (Connectoren) mit gasdichten Glas- oder Keramikisolatoren ausgeführt. Der Modulare Aufbau mit Verbindern oder Konnektoren ermöglicht ein Aneinanderfügen der Arrays zur flexiblen Anpassung an branchenübliche Bioreaktordimensionen und unterstützt hiermit auch den Up-Scale.

Dabei kann das Gehäuse modular anreihbar ausgebildet sein und insbesondere Befestigungsmittel zur modularen Anreihung umfassen.

Es ist von Vorteil, wenn innerhalb des Gehäuses der Vorrichtung zur Abgabe elektromagnetischer Strahlung ein gegenüber dem Äußeren des Gehäuses verminderter Druck herrscht und insbesondere das Gehäuse einen fluiddicht verschließbaren Evakuierungsport aufweist, denn dann kann das Gehäuse mittels des Evakuierungsports evakuiert werden, so dass dieses einen gegenüber dessen Äußerem verminderten Druck aufweist und hierdurch Wärmeleitung vom Gehäuse der Vorrichtung zur Abgabe elektromagnetischer Strahlung zu darin angeordneten Baugruppen vermindert werden kann.

Hierdurch können insbesondere elektronische Baugruppen längere Zeiten ausfallfrei überstehen, während welchen das Gehäuse, beispielsweise bei einer Sterilisierung oder Autoklavierung ausgesetzt ist, überstehen.

In überraschender Weise konnte festgestellt werden, dass die hier offenbarten Vorrichtungen zur Abgabe elektromagnetischer Strahlung eine Anzahl von mehr als 3500 Autoklavierungszyklen ausfallfrei überstanden haben. Autoklavierbar wird im Rahmen dieser Offenbarung auch als autoklavierbar im Sinne der DIN EN ISO 14937; EN ISO 17665, welche für Medizinprodukte gültig ist, verstanden.

Ein bevorzugtes Verfahren zur Vermehrung oder Kultivierung biologischen Materials umfasst das Einbringen biologischen Materials oder einer Vorstufe biologischen Materials in einen hier beschriebenen Photobioreaktor, das Einwirkenlassen elektromagnetischer Strahlung, welche von einer Vorrichtung zur Abgabe elektromagnetischer Strahlung, wie diese hier beschrieben wird, emittiert wurde.

Vorteilhaft umfasst ein Verfahren zum Präparieren von biologischem Material und/oder Herstellen von Pharmazeutika, insbesondere Biopharmazeutika das Bereitstellen eines Photobioreaktors, der mit zumindest einer Vorrichtung zur Abgabe elektromagnetischer Strahlung, wie diese vorliegend offenbart wird, bestückt wird.

Wenn der Photobioreaktor zumindest eine Durchführung aufweist, insbesondere zumindest einen Standard-Sparger-Port, wobei Zuleitungen und/oder Kontrollleitungen für die Vorrichtung zur Abgabe elektromagnetischer Strahlung durch die zumindest eine Durchführung geführt werden, können oft auch herkömmliche Photobioreaktoren oder auch herkömmliche Bioreaktoren mit der hier offenbarten Vorrichtung zur Abgabe elektromagnetischer Strahlung auf einfache Weise und kostengünstig nachgerüstet werden.

Besonders günstig ist es hierbei, wenn der Photobioreaktor in seinem Innern, insbesondere dem Inneren des Behälters zur Aufnahme biologischen Materials zumindest eine Montageeinrichtung zum Fixieren von Prall- oder Verwirbelungsblechen aufweist und die zumindest eine Vorrichtung zur Abgabe elektromagnetischer Strahlung mit zumindest dieser einen Montageeinrichtung verbunden wird, wobei bevorzugt die Vorrichtung zur Abgabe elektromagnetischer Strahlung durch die zumindest eine Durchführung und die zumindest eine Montageeinrichtung im Innern des Photobioreaktors, insbesondere dem Inneren des Behälters zur Aufnahme biologischen Materials fixiert wird.

Für Single-Use-Anwendungen kann die Vorrichtung zur Abgabe elektromagnetischer Strahlung eine magnetische und/oder adhäsive Fixierung mit induktiver Energieeinkopplung und Signalübertragung aufweisen.

Ein für den Anwender besonderes günstiger Vorteil entsteht, wenn der Photobioreaktor nach Bestückung mit der Vorrichtung zur Abgabe elektromagnetischer Strahlung sterilisiert wird. Hierdurch kann eine Kontamination des Photobioreaktors nach dessen Sterilisierung weitestgehend vermieden werden.

Weiterhin vorteilhaft ist es, wenn die Vorrichtung zur Abgabe elektromagnetischer Strahlung zumindest einen Sensor aufweist und mittels des Sensors im Betriebszustand bevorzugt die Intensität und/oder Wellenlänge von einfallender elektromagnetischer Strahlung messbar und/oder einstellbar, insbesondere geregelt einstellbar ist und im Betriebszustand die Strahlungsintensität und/oder Wellenlänge der elektromagnetischen Strahlung im Innern des Bioreaktors mit diesem Sensor gemessen wird.

Wenn für einen definierten Zeitraum elektromagnetische Strahlung einer definierten Wellenlänge, bevorzugt 250 nm in den Photobioreaktor eingestrahlt wird und nach dieser Einstrahlung breitbandig eine Strahlungsintensität und/oder Wellenlänge der elektromagnetischen Strahlung im Innern des Bioreaktors gemessen wird oder selektiv bei einer Wellenlänge, insbesondere bei 270 nm eine Strahlungsintensität der elektromagnetischen Strahlung im Innern des Bioreaktors gemessen wird, können hierdurch fluoreszent emittierte Anteile des Lichts detektiert und bezüglich definierter Stoffwechselvorgänge innerhalb des Photobioreaktors ausgewertet werden.

Ferner kann besonders vorteilhaft, beispielsweise während einer Produktionsphase für einen definierten Zeitraum elektromagnetische Strahlung einer definierten Wellenlänge, von bevorzugt 250 nm in den Photobioreaktor eingestrahlt werden und nach dieser Einstrahlung elektromagnetische Strahlung einer definierten weiteren Wellenlänge, bevorzugt in einem Bereich von 620 bis 780 nm in den Photobioreaktor eingestrahlt werden.

Es ist von Vorteil, wenn bei einer Ausführungsform des Verfahrens der Photobioreaktor mit dessen Behälter zur Aufnahme von fluiden Medien, die biologisches Material enthalten, und der Vorrichtung zur Abgabe elektromagnetischer Strahlung autoklaviert wird während die Vorrichtung zur Abgabe elektromagnetischer Strahlung mit deren Gehäuse innerhalb des Behälters zur Aufnahme von biologischem Material angeordnet ist, denn dann können Kontaminationen des Photobioreaktors mit sehr viel höherer Wahrscheinlichkeit als bisher vermieden werden und kann die Autoklavierung mit einem einzigen Vorgang durchgeführt werden. Wird folglich die Autoklavierung zeitnahe vor der Befüllung des Photobioreaktors mit dem biologischen Material vorgenommen, reduziert sich auch das Risiko des zwischenzeitlichen Eintrags von Verschmutzungen.

Bei den hier offenbarten Verfahren können insbesondere auch durch Mutagenese veränderte photo- oder mixotrophen Mikroorganismen, insbesondere auch Mikroalgen, Hefen und Bakterien, verwendet werden.

In dem Artikel "A novel histone crosstalk pathway important for regulation of UV-induced DNA damage repair in Saccharomyces cerevisiae", Boudoures, A.L., Pfeil, J.J., Steenkiste, E.M., Hoffman, R.A., Bailey, E.A., Wilkes, S.E., Higdon, S.K., Thompson, J.S., (2017) Genetics, 206 (3), pp. 1389-1402 wird über zelluläre Reperaturmechanismen berichtet, welche erhöhte Erträge bei der Vermehrung biologischen Materials erbringen können.

Ein bevorzugter genereller Mechanismus kann hierbei die Destabilisierung von Aminen bei bestimmten eingestrahlten Wellenlängen sein, welcher durch entsprechende Reparaturmechanismen zu erhöhten Erträgen der jeweils betroffenen Zelle führt.

Diesen Mechanismen kann mit der vorliegenden Einrichtung entsprechend Rechnung getragen werden, indem beispielsweise elektromagnetische Strahlung mit einer Wellenlänge von 250 nm in den Behälter zur Aufnahme biologischen Materials eingestrahlt wird, wie dieses nachfolgend noch detaillierter beschrieben werden wird.

Ein weiteres Beispiel für die Beeinflussbarkeit von Signalkaskaden ist in "Inhibitory effects of ginsenosides on basic fibroblast growth factorinduced melanocyte proliferation", Lee, J.E., Park, J.I., Myung, C.H., Hwang, J.S., (2017) Journal of Ginseng Research, 41 (3), pp. 268-276.

Zum einen wird angeführt, dass durch UV-B die Proliferation stimuliert wird, welches ebenfalls basierend auf den hier beschriebenen Verfahren erfolgen kann.

Zum anderen kann eine Inhibierung durch eine Antikörperzugabe (potentiell durch einen feed -Kanal des Bioreaktors) bewirken werden und es lässt sich mittels einer Sensoreinheit insbesondere den hier beschriebenen Sensoren, die Aktivierung über ausgewählte Wellenlängen des LED-Arrays und Inhibierung durch den ansprechenden Feed, erfassen.

In "ROS and calcium signaling mediated pathways involved in stress responses of the marine microalgae Dunaliella salina to enhanced UV-B radiation", Zhang, X., Tang, X., Wang, M., Zhang, W., Zhou, B., Wang, Y. (2017) Journal of Photochemistry and Photobiology B: Biology, 173, pp. 360-367, wird beschrieben, dass mit steigender UV-B Intensität die Lyse von Microalgen steigt. Diese Verringerung der Zelldichte geht mit einer Erhöhung von bestimmten Proteinen einher, wodurch sich die Produktbildung dieser Proteine gezielt mit den hier beschriebenen Vorrichtungen und Verfahren steuern und erhöhen lässt.

In "Model-supported phototrophic growth studies with Scenedesmus obtusiusculus in a flat-plate photobioreactor", Koller, A.P., Löwe, H., Schmid, V., Mundt, S., Weuster-Botz, D., (2017) Biotechnology and Bioengineering, 114 (2), pp. 308-320., wird eine parameterabhängige Wachstumskinetik beschrieben. Als Parameter werden Lichtstromintensität, Zelllinien abhängige Adsorption und Streuung, sowie indirekt die Kultivierungszeit beschrieben. Eine, durch Optimierung der Strahlungsintensität, gesteigerte Biomassenausbeute wird angegeben. Dies bestätigt die Vorteile eines Regelkreises von LED-Arrays und einer Sensoreinheit, wie diese vorliegend offenbart werden.

Die beschriebenen Ausführungsformen ermöglichen insbesondere auch eine mixotrophe Kultivierung und es lässt sich vorteilhaft Photosynthese und Chemosynthese kombinieren. Es wird dabei auch eine sterile und verbesserte Produktion von Pharmazeutika mit Mikroalgen ermöglicht.

Der Artikel "Adhesion of Chlamydomonas microalgae to surfaces is switchable by light", Christian Titus Kreis, Marine Le Blay, Christine Linne, Marcin Michal Makowski and Oliver Bäumchen, NATURE PHYSICS DOI: 10.1038/NPHYS4258 beschreibt das Verhalten von Mikroalgen, welche mittels der Einstrahlung von Licht dazu gebracht werden konnten, sich an Oberflächen anzuhaften oder von Oberflächen zu lösen. Durch gezielte Einstrahlung elektromagnetischer Strahlung entsprechender Wellenlänge kann nun beispielsweise für Photobioreaktoren, welche im repeated Batch-Betrieb eingesetzt werden, erreicht werden, dass sich während des Wechsels eines Mediums, beispielsweise von Nährlösung, diese Mikroalgen an Oberflächen anhaften und nach Zufuhr des Mediums sich wieder von der Oberfläche lösen. Auch hierdurch kann die Effektivität des Betriebs von Photobioreaktoren weiter gesteigert werden.

Die Autoren Ni, M., Tepperman, J.M. and Quail, P.H. (1999) beschreiben in der Veröffentlichung (Phytochromes and gene expression), "Binding of phytochrome B to its nuclear signalling partner PIF3 is reversibly induced by light, Nature 400, 781-784, dass sich die Genexpression über die entsprechenden Zellsignalwege gezielt lichtabhängig induzieren lässt.

Die Veröffentlichung "A red/far-red light-responsive bistable toggle switch to control gene expression in mammalian cells", Konrad Müller, Raphael Engesser, Stephanie Metzger, Simon Schulz, Michael M. Kämpf, Moritz Busacker, Thorsten Steinberg, Pascal Tomakidi, Martin Ehrbar, Ferenc Nagy, Jens Timmer, Matias D. Zubriggen und Wilfried Weber, Nucleic Acids Research, 2013, Vol. 41, No. 7 e77, beschreibt diesen Mechanismus für Mammalia-Zellen, um transgene Aktivität zu kontrollieren. Optogenetische Kontrolle ist auch in beschrieben in "A Phytochrome-Derived Photoswitch for Intracellular Transport", Max Adrian, Wilco Nijenhuis, Rein I. Hoogstraaten, Jelmer Willems, and Lukas C. Kapitein, DOI: 10.1021/acssynbio.6b00333 ACS Synth. Biol. 2017, 6, 1248-1256. Wellenlängenspezifisch lassen sich gemäß diesem Dokument intrazelluläre bidirektionale Transportmechanismen steuern.

Die Erfindung wird nachfolgend unter Bezugnahme auf die beigefügten Zeichnungen anhand bevorzugter Ausführungsformen detaillierter beschrieben.

Es zeigen:
- Figur 1: eine schematische Querschnittsansicht, welche durch die Mitte eines Photobioreaktors einer ersten bevorzugten Ausführungsform in vertikaler Richtung in der in Figur 8 dargestellten Schnittebene AA verläuft, und bei welcher ein oberer, den Behälter zur Aufnahme biologischen Materials nach oben hin abschließender Abschnitt um der Klarheit Willen stark vereinfacht dargestellt ist,
- Figur 2: eine schematische Seitenansicht eines Photobioreaktors der bevorzugten ersten Ausführungsform, bei welcher ein oberer, den Behälter zur Aufnahme biologischen Materials nach oben hin abschließender Abschnitt um der Klarheit Willen stark vereinfacht dargestellt ist,
- Figur 3: eine schematische Querschnittsansicht, welche horizontal durch den Photobioreaktor der ersten bevorzugten Ausführungsform in der in Figur 2 dargestellten Schnittebene BB verläuft,
- Figur 4: eine Querschnittsansicht, welche horizontal durch den Photobioreaktor der ersten bevorzugten Ausführungsform in der in Figur 2 dargestellten Schnittebene CC verläuft,
- Figur 5: eine detailliertere und vergrößerte Darstellung eines Ausschnitts aus Figur 4, welcher jedoch relativ zu Figur 4 leicht gedreht gezeigt ist mit dem Sparger-Port und einer mittels einer Durchführungs- und Halteeinrichtung an dieser gehaltenen Vorrichtung zur Abgabe elektromagnetischer Strahlung,
- Figur 6: eine Darstellung eines schräg stehend angeordneten Photobioreaktors der ersten bevorzugten Ausführungsform, schräg von oben, bei welcher der obere, den Behälter zur Aufnahme biologischen Materials nach oben hin abschließende Abschnitt um der Klarheit Willen nicht und der Behälter zur Aufnahme biologischen Materials transparent dargestellt ist,
- Figur 7: eine schematische Schnittdarstellung eines Abschnitts eines aufrecht stehend angeordneten Photobioreaktors der ersten bevorzugten Ausführungsform, schräg von oben, bei welcher der obere, den Behälter zur Aufnahme biologischen Materials nach oben hin abschließende Abschnitt um der Klarheit Willen nicht und der Behälter zur Aufnahme biologischen Materials transparent dargestellt ist,
- Figur 8: nochmals eine vergrößerte Darstellung der Figur 4, welche jedoch relativ zu Figur 4 leicht gedreht gezeigt ist, zur Beschreibung der emittierten elektromagnetischen sowie gemessenen elektromagnetischen Strahlung,
- Figur 9: eine horizontal durch die Vorrichtung zur Abgabe elektromagnetischer Strahlung verlaufende schematische Schnittdarstellung und ein Detail des Photobioreaktors, schräg von oben, bei welcher die Schnittebene im Wesentlichen der in Figur 3 dargestellten Schnittebene entspricht und der Behälter zur Aufnahme biologischen Materials transparent dargestellt ist,
- Figur 10: eine schematische Querschnittsansicht, welche durch die Mitte eines Photobioreaktors der ersten bevorzugten Ausführungsform in vertikaler Richtung in der in Figur 8 dargestellten Schnittebene DD verläuft, und bei welcher ein oberer, den Behälter zur Aufnahme biologischen Materials nach oben hin abschließender Abschnitt um der Klarheit Willen nicht und der Behälter zur Aufnahme biologischen Materials transparent dargestellt ist,
- Figur 11: ein Detail der Querschnittsdarstellung der Figur 10, welches in Figur 10 mit dem Bezugszeichen E versehen ist,
- Figur 12: ein Detail der Querschnittsdarstellung der Figur 10, welches in Figur 10 mit dem Bezugszeichen F versehen ist,
- Figur 13: eine Ausführungsform einer Quelle zur Abgabe elektromagnetischer Strahlung, welche einen zweizeiligen Array von LEDs und einen zweizeiligen Array von Sensoren sowie zwei Controller zur Ansteuerung der LEDs und zur Kommunikation mit den Sensoren umfasst,
- Figur 14: eine Ausführungsform einer Quelle zur Abgabe elektromagnetischer Strahlung, welche einen einzeiligen Array von LEDs und einen einzeiligen Array von Sensoren sowie einen Controller zur Ansteuerung der LEDs und zur Kommunikation mit den Sensoren umfasst,
- Figur 15: eine entlang der Symmetrieachse S verlaufende schematische Schnittdarstellung eines hier beschriebenen Fensters zur Erläuterung von dessen Herstellungsverfahren,
- Figur 16: eine entlang der Symmetrieachse S eines Fensters der Vorrichtung zur Abgabe elektromagnetischer Strahlung verlaufende schematische Schnittdarstellung mit einem Teil des Gehäusekörpers der Vorrichtung zur Abgabe elektromagnetischer Strahlung und einem planparallelen transparenten Element, wobei die Symmetrieachse S lediglich beispielhaft für ein Fenster in Figur 9 dargestellt ist,
- Figur 17: eine entlang der Symmetrieachse S eines Fensters der Vorrichtung zur Abgabe elektromagnetischer Strahlung verlaufende schematische Schnittdarstellung mit einem Teil des Gehäusekörpers der Vorrichtung zur Abgabe elektromagnetischer Strahlung und einem bikonvexen transparenten Element,
- Figur 18: eine entlang der Symmetrieachse S eines Fensters der Vorrichtung zur Abgabe elektromagnetischer Strahlung verlaufende schematische Schnittdarstellung mit einem Teil des Gehäusekörpers der Vorrichtung zur Abgabe elektromagnetischer Strahlung und einem bikonkaven transparenten Element,
- Figur 19: eine entlang der Symmetrieachse S eines Fensters der Vorrichtung zur Abgabe elektromagnetischer Strahlung verlaufende schematische Schnittdarstellung mit einem Teil des Gehäusekörpers der Vorrichtung zur Abgabe elektromagnetischer Strahlung und einem plankonvexen transparenten Element,
- Figur 20: eine entlang der Symmetrieachse S eines Fensters der Vorrichtung zur Abgabe elektromagnetischer Strahlung verlaufende schematische Schnittdarstellung mit einem Teil des Gehäusekörpers der Vorrichtung zur Abgabe elektromagnetischer Strahlung und einem plankonkaven transparenten Element,
- Figur 21: eine entlang der Symmetrieachse S eines Fensters der Vorrichtung zur Abgabe elektromagnetischer Strahlung verlaufende schematische Schnittdarstellung mit einem Teil des Gehäusekörpers der Vorrichtung zur Abgabe elektromagnetischer Strahlung und einem plankonkaven transparenten Element
- Figur 22: eine entlang der Symmetrieachse S eines Fensters der Vorrichtung zur Abgabe elektromagnetischer Strahlung verlaufende schematische Schnittdarstellung mit einem Teil des Gehäusekörpers der Vorrichtung zur Abgabe elektromagnetischer Strahlung und einem plankonkaven transparenten Element,
- Figur 23: eine entlang der Symmetrieachse S verlaufende schematische Schnittdarstellung eines hier beschriebenen Fensters, bei welchem dessen transparentes Element Quarzglas umfasst, zur Erläuterung von dessen Herstellungsverfahren,
- Figur 24: eine Aufsicht auf das in Figur 23 dargestellte Fester, bei welchem dessen transparentes Element Quarzglas umfasst,
- Figur 25: einen Querschnitt durch einen Teil des Gehäuses des oberen oder ersten Moduls der Vorrichtung zur Abgabe elektromagnetischer Strahlung oder auch des Gehäuses des unteren oder zweiten Moduls der Vorrichtung zur Abgabe elektromagnetischer Strahlung.

Die Erfindung wird nachfolgend anhand bevorzugter Ausführungsformen detaillierter beschrieben.

Bei den Figuren, welche um der Klarheit Willen nicht maßstabsgerecht dargestellt sind, bezeichnen gleiche Bezugszeichen jeweils gleiche oder gleichwirkende Bestandteile.

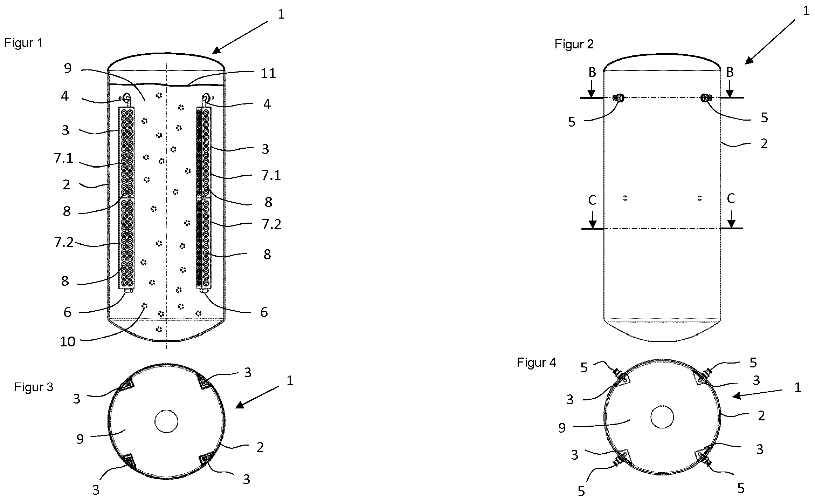

Nachfolgend wird auf die Figuren 1 und 2 Bezug genommen, in welcher jeweils ein aufrecht stehender Photobioreaktor 1 in dessen bevorzugter Betriebsstellung einer bevorzugten ersten Ausführungsform gezeigt ist.

Alle nachfolgenden Positionsangaben beziehen sich auf diese Stellung des Photobioreaktors und Begriffe wie oben, unten, neben, oberhalb oder unterhalb orientieren sich im Rahmen der vorliegenden Offenbarung an dieser Stellung des Photobioreaktors.

Der Photobioreaktor 1 umfasst einen Behälter 2 zur Aufnahme von fluiden Medien, die biologisches Material enthalten, welcher nachfolgend auch abkürzend als Behälter 2 zur Aufnahme von biologischem Material oder auch nur als Behälter 2 bezeichnet wird.

Der Behälter 2 der bevorzugten Ausführungsformen besteht aus Edelstahl oder umfasst Edelstahl. Der Edelstahl kann 316L Pharmastahl umfassen, oder vollständig aus diesem bestehen.

Innerhalb des Behälters zur Aufnahme von biologischem Material 2 ist zumindest eine Vorrichtung 3 zur Abgabe elektromagnetischer Strahlung mit deren Gehäuse 7 an deren oberen Ende mittels einer Durchführungs- und Halteeinrichtung 4 an einem Sparger-Port, vorzugsweise einem Standart-Sparger-Port 5 gehalten.

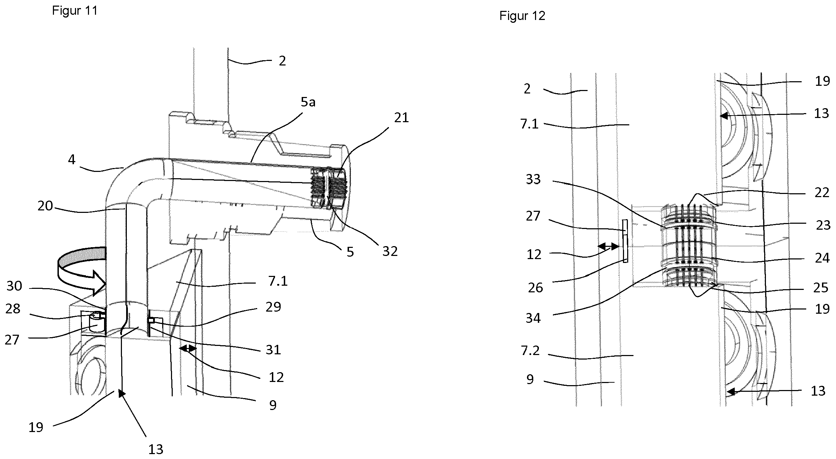

Die einem Fachmann auf diesem Gebiet bekannten Sparger-Ports 5 bilden Durchführungen 5a, welche durch zumindest eine Wand des Photobioreaktors, insbesondere durch die Wand des Behälters 2 führen, wie dieses nachfolgend noch detaillierter und unter Bezugnahme auf Figur 11 beschrieben werden wird. Hierdurch wird die Vorrichtung zur Abgabe elektromagnetischer Strahlung 3 an zumindest einer Durchführung des Photobioreaktors gehalten.

Am unteren Ende der Vorrichtung 3 zur Abgabe elektromagnetischer Strahlung ist diese jeweils mit zumindest einer Montageeinrichtung 6 an der Innenwand des Behälters 2 gehalten. Die Montageeinrichtung 6 kann eine Montageeinrichtung sein, wie diese in üblicher Weise zum Fixieren, insbesondere von Prall- oder Verwirbelungsblechen dient und in herkömmlicher Weise in Bio- oder Photobioreaktoren verwendet wird, beispielsweise ein Inforce HT-Adapter, oder kann eine eigene standardisierte Aufnahme zur Halterung umfassen, wie beispielsweise eine mit einer Durchgangsbohrung versehende Halterung, welche an einem vom Behälter 2 des Photobioreaktors hervorstehenden Bolzen gehalten ist. Lediglich beispielhaft ist jeweils eine Montageeinrichtung 6 mit deren Durchgangsbohrung in Figur 1 angedeutet.

Die Vorrichtung 3 Abgabe elektromagnetischer Strahlung umfasst ferner ein Gehäuse 7 und bei der bevorzugten ersten Ausführungsform ein Gehäuse 7.1 eines oberen Moduls und ein Gehäuse 7.2 eines unteren Moduls, welches jeweils zumindest ein Fenster 8 für den Durchtritt elektromagnetischer Strahlung in das Gehäuse 7.1, 7.2 oder aus dem Gehäuse 7.1, 7.2 heraus aufweisen.

Lediglich beispielhaft ist in Figur 1 nur jeweils ein Fenster 8 mit einem Bezugszeichen versehen dargestellt; zu erkennen ist jedoch, dass in dem Gehäuse 7.1 und 7.2 jeweils eine Vielzahl von Fenstern 8 angeordnet ist.

Bei einer weiteren bevorzugten Ausführungsform umfasst die Vorrichtung 3 zur Abgabe elektromagnetischer Strahlung jeweils statt zwei Modulen mit jeweils einem Gehäuse 7.1, 7.2 nur ein Gehäuse, welches die Länge und Anzahl an Fenstern beider der in Figur 1 dargestellten Module aufweist oder welches alternativ auch kleinere Abmessungen in Längsrichtung und eine geringere Anzahl von Fenstern aufweisen kann. In weiterer Ausgestaltung der Erfindung kann die Vorrichtung 3 auch mehr als 2 Module umfassen.

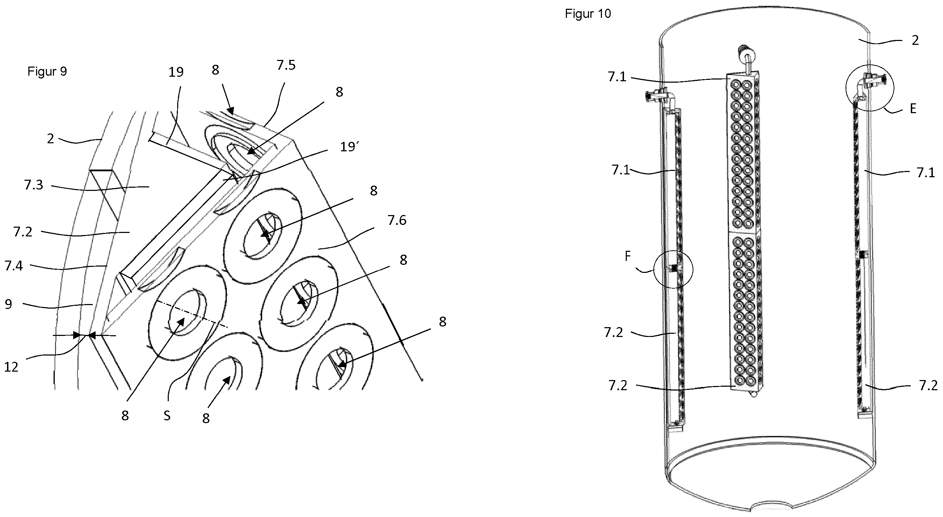

Wie besonders gut den Figuren 9, 11 und 12 zu entnehmen, ist die Vorrichtungen 3 zur Abgabe elektromagnetischer Strahlung mit deren Gehäuse 7, 7.1, 7.2 beabstandet zu der Wand, insbesondere Innenwand des Behälters 2 zur Aufnahme von biologischem Material angeordnet. Hierbei besteht zwischen dem Gehäuse 7, 7.1, 7.2 und der Wand des Behälters 2 jeweils ein in diesen Figuren mit einem Doppelpfeil dargestellter minimaler Abstand 12, welcher sicherstellt, dass fluide Material 9, welches biologisches Material 10 enthält, die Gehäuse 7, 7.1 und 7.2 der Vorrichtung 3 allseitig umströmen kann, wodurch die Vorrichtung 3 jeweils die Funktion eines Prall- oder Verwirbelungsbleches übernehmen kann.

Zu erkennen ist ebenfalls, dass die Vorrichtung 3 in deren bevorzugter Stellung vollständig in das fluide Medium 9, welches biologisches Material 10 enthält, eingetaucht ist, denn die Oberfläche 11 des fluiden Mediums 9 liegt bevorzugt zumindest oberhalb der Fenster 8.

Das biologische Material, welches lediglich beispielhaft in Figur 1 einmalig mit dem Bezugszeichen 10 und um der Erkennbarkeit Willen sehr stark vergrößert dargestellt ist, umfasst generell photo-, hetero- und mixotrophen Mikroorganismen, pro- und eukaryotische Zellen, bei denen Photonen Zellsignalwege triggern und liegt beispielsweise in Form von Mikroalgen, beispielsweise Blaualgen oder Bakterien, wie beispielsweise Cyanobakterien vor und umfasst insbesondere auch durch Mutagenese veränderten photo- oder mixotrophe Mikroorganismen oder auch Hefen.

Das fluide Material kann als wässrige Lösung vorliegen und Nährstoffe für das biologische Material, wie beispielsweise Zucker umfassen.

Nachfolgend wird auf die Schnittdarstellung der Figur 3 Bezug genommen, in welcher zu erkennen ist, dass innerhalb des Behälters 2 zur Aufnahme von biologischem Material mehrere, insbesondere bei der ersten bevorzugten Ausführungsform vier Vorrichtungen 3 zur Abgabe elektromagnetischer Strahlung, angeordnet sind. Diese sind jeweils symmetrisch zur Längsachse des Behälters 2 angeordnet.

Der Querschnittsansicht von Figur 4, ist die entsprechende Lage der Sparger-Ports 5, welche jeweils Durchführungen 5a durch die Wand des Behälters 2 des Photobioreaktors 1 bilden zu entnehmen, gut zu entnehmen. Mittels einer Durchführungs- und Halteeinrichtung 4, welche nachfolgend noch detaillier unter Bezugnahme auf Figur 11 beschrieben wird, ist die Vorrichtung 3 zur Abgabe elektromagnetischer Strahlung jeweils an der Wand des Behälters 2 gehalten und kann diese hierdurch auch bei herkömmlichen Photobioreaktoren, welche derartige Sparger-Ports aufweisen, nachgerüstet werden.

Aus der Schnittdarstellung der Figur 11 ist zu erkennen, dass sich die im Wesentlichen L-förmige und hohl vorzugsweise rohrförmig ausgeführte Durchführungs- und Halteeinrichtung 4 durch die von den Sparger-Ports 5 ausgebildeten Durchführungen 5a erstrecken und an deren der Außenseite des Photobioreaktors zugewandter Seite einen Verbinder 21 umfassen kann.

Die Durchführungs- und Halteeinrichtung 4 ist in den Sparger-Ports 5 und somit in den von diesen gebildeten Durchführungen 5a mit entsprechenden, dem Fachmann bekannten Dichtmitteln, beispielsweise O-Ringen abgedichtet gehalten.

Hierdurch wird eine Nachrüstung und ein Austausch der Vorrichtung 3 zur Abgabe elektromagnetischer Strahlung ermöglicht, indem die Durchführungs- und Halteeinrichtung 4 mit daran befestigter Vorrichtung 3 in den Sparger-Port 5 eingesetzt oder diesem entnommen wird.

Für eine permanente Befestigung der Vorrichtung 3 zur Abgabe elektromagnetischer Strahlung kann die Durchführungs- und Halteeinrichtung 4 auch beispielsweise mittels Löten unter Verwendung eines Goldlotes dauerhaft und hermetisch dicht mit dem Sparger-Port 5 verbunden werden.

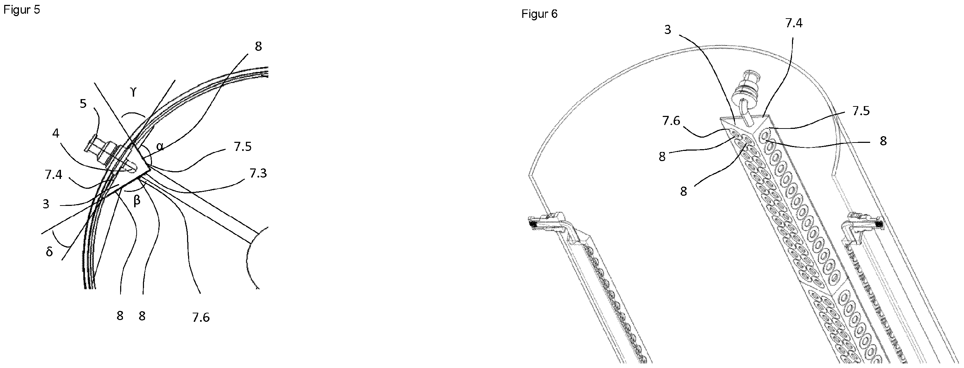

Nachfolgend wird auf Figur 5 Bezug genommen, welche eine detailliertere und vergrößerte Darstellung eines Ausschnitts aus Figur 4 zeigt, der jedoch relativ zu Figur 4 leicht gedreht dargestellt ist.

Es ist zu erkennen, dass die in einer Aufsicht dargestellte Vorrichtung 3 zur Abgabe elektromagnetischer Strahlung an dem Sparger-Port 5 mittels einer Durchführungs- und Halteeinrichtung 4 gehalten ist.

Ferner ist dieser Darstellung auch der dreieckförmige Querschnitt der säulenförmigen Vorrichtung 3 zu entnehmen.

Das Gehäuse 7, 7.1, 7.2 bildet jeweils mit dessen säulenförmig ausgebildetem Gehäusekörper 7.3 drei, sich in dessen Längsrichtung erstreckende Seitenwände 7.4, 7.5 und 7.6 aus. Bevorzugt kann der Gehäusekörper 7.3 jeweils aus einem oder aus mehreren Frästeilen ausgebildet sein.

Der Gehäusekörper 7.3 besteht bevorzugt aus Edelstahl oder umfasst zumindest Edelstahl.

Verwendbar sind hierbei alle Edelstähle, insbesondere auch austenitische und ferritische Edelstähle, bevorzugt jedoch nur soweit diese bei der Durchführung der Erfindung rostfrei bleiben.

Der Edelstahl kann bevorzugt auch 316L Pharmastahl umfassen, oder vollständig aus diesem bestehen.

Ferner sind prinzipiell auch Titan sowie Monell-Legierung mit hohem Kupferanteil verwendbar, wobei das Material bei Verwendung für die Vorrichtung zur Abgabe elektromagnetischer Strahlung, insbesondere für deren Gehäusekörper auch emailliert sein kann.

Alternativ kann der Gehäusekörper 7.3 auch einen hochtemperaturbeständigen Kunststoff, insbesondere einen thermoplastischen Kunststoff wie einen Polyaryletherketon, insbesondere einen Polyetheretherketon, PEEK, umfassen oder aus diesem bestehen.

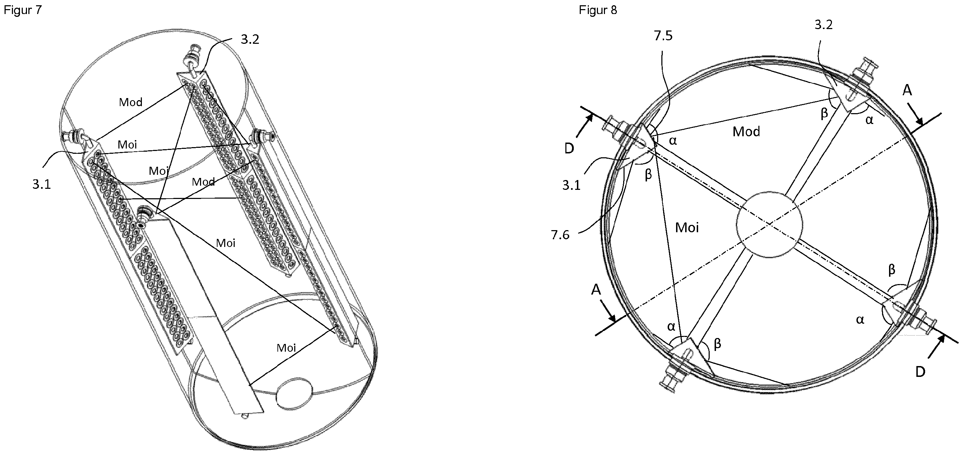

Zumindest zwei der sich in Längsrichtung erstreckenden Seitenwände 7.4, 7.5 und 7.6 umfassen jeweils Fenster 8, wie dieses auch der Darstellung der Figuren 6 und 7 besonders gut zu entnehmen ist. Diese beiden Seitenwände sind beispielhaft die Seitenwände 7.5 und 7.6, wie dieses auch in den Figuren 6 und 7 jeweils gezeigt ist.

Zwei der sich in Längsrichtung erstreckenden Seitenwände 7.4, 7.5 und 7.6 weisen einen 30° Winkel und zwei von dessen sich in Längsrichtung erstreckenden Seitenwänden 7.4, 7.5 und 7.6 einen 60° Winkel zueinander auf.

In Abhängigkeit von einem jeweils verwendeten Rührorgan sowie auch von der Anzahl der hier beschriebenen Vorrichtungen zur Abgabe elektromagnetischer Strahlung können auch vorteilhaft andere Winkel als 30° Winkel und 60° Winkel verwendet werden.

Die vorstehend angegebenen Winkel können auch speziell an vorhandene Rührorgane angepasst werden und dann andere Beträge aufweisen.

In der in Figur 5 gezeigten Ausführungsform weisen die Seitenwände 7.4 und 7.5 den Winkel Y zueinander auf, welcher 60° beträgt und weisen die Seitenwände 7.4 und 7.6 den Winkel δ zueinander auf, welcher 30° beträgt.

Durch diese Wahl der Winkel Y und δ wird eine besonders homogene Ausleuchtung des Behälters 2 ermöglicht, wie dieses an späterer Stelle noch unter Bezugnahme auf Figur 8 detaillierter erläutert werden wird.

Dennoch ist diese Wahl der Winkel Y und δ nicht als beschränkend zu verstehen, da auch andere Winkel, beispielsweise bei weniger als vier oder mehr als vier Vorrichtungen 3 zur Abgabe elektromagnetischer Strahlung in einem Behälter 2 vorteilhaft sein können.

In dem Gehäuse 7, 7.1m 7.2 der Vorrichtung 3 zur Abgabe elektromagnetischer Strahlung ist jeweils vorzugsweise zumindest eine Quelle 13 zur Abgabe elektromagnetischer Strahlung und ist bevorzugt jeweils zumindest ein Sensor 14 zum Messen elektromagnetischer Strahlung angeordnet, welche nachfolgend unter Bezugnahme auf die Figuren 9, 13 und 14 detaillierter beschrieben werden.

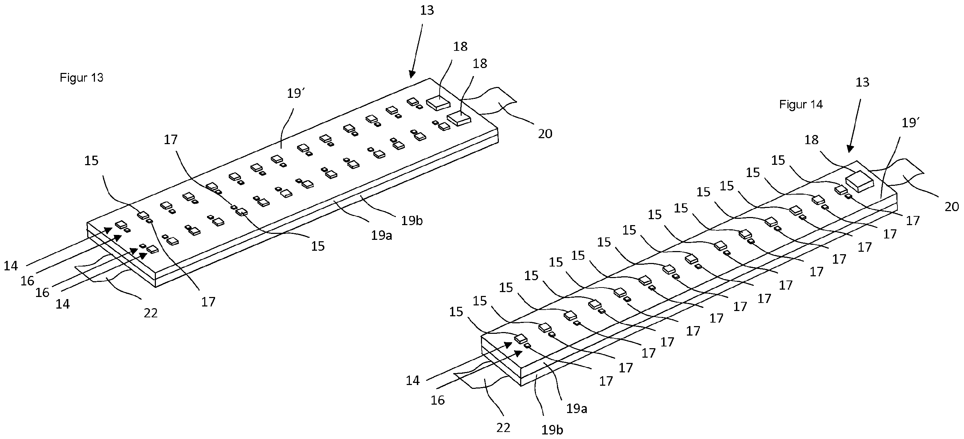

Figur 14 zeigt dabei eine Ausführungsform einer Quelle 13 zur Abgabe elektromagnetischer Strahlung, welche einen einzeiligen Array 14 von LEDs 15 und einen einzeiligen Array 16 von Sensoren 17 sowie einen Controller 18 zur Ansteuerung der LEDs 15 und zur Kommunikation mit den Sensoren 17 umfasst.

Sowohl die LEDs 15 des Arrays 14 als auch die Sensoren 17 des Arrays 16 sind jeweils mittels einer gedruckten Schaltungsplatine (PCB) 19 mit dem Controller 18 leitend verbunden und an der Vorderseite der jeweiligen gedruckten Schaltungsplatine (PCB) w19, 19' angebracht. Da die leitenden Verbindungen der Schaltungsplatine (PCB) 19 innerhalb der Platine 19 verlaufen, sind diese in den Figuren nicht dargestellt, jedoch dem Fachmann an sich bekannt. Die Rückseite der Schaltungsplatinen 19, 19' weisen in der Darstellung der Figuren 13 und 14 jeweils in die Richtung des Papiers, auch welchem diese dargestellt sind.

Figur 13 zeigt eine weitere Ausführungsform einer Quelle 13 zur Abgabe elektromagnetischer Strahlung, welche gegenüber der Figur 14 gezeigten Ausführungsform einen zweizeiligen Array 14 von LEDs 15 und einen zweizeiligen Array 16 von Sensoren 17 sowie zwei Controller 18 zur Ansteuerung der LEDs 15 und zur Kommunikation mit den Sensoren 17 umfasst, aber ansonsten der Ausführungsform der Figur 13 funktional im Wesentlichen gleicht. Hierbei umfasst diese Ausführungsform eine gedruckte Schaltungsplatine (PCB) mit dem Bezugszeichen 19', um diese Schaltungsplatine (PCB) von der in Figur 14 dargestellten Schaltungsplatine mit dem Bezugszeichen 19 unterscheiden zu können.

Es kann beispielsweise für die Schaltungsplatine 19' ein leitend bedrucktes Mehrlagenpaket mit keramischen Substraten 19a und 19b mit sehr hoher thermischer Beständigkeit und auch mit hoher elektrischer Leitfähigkeit bereitgestellt werden.

Als Keramiksubstrat 19a, 19b wird gemäß einer bevorzugten Ausführungsform der Erfindung ein DCB (direct copper bond)-Keramiksubstrat verwendet.

Es handelt sich dabei um ein Keramiksubstrat, wobei auf einen keramischen Isolator in einem Hochtemperaturschmelz-und Diffusionsprozess Kupfer, insbesondere reines Kupfer, aufgebracht wird und so haftfest mit der Keramik verbunden ist. Neben einer festen Verbindung mit dem Substrat können so verhältnismäßig dicke Leiterbahnen, insbesondere Leiterbahnen mit einer Dicke von über 200 µm, aufgebracht werden. Über die Leiterbahnen eines DCB-Substrats lassen sich so große Ströme leiten.

Bei einer anderen Ausführungsform wird ein DPC (direct plated copper) Keramiksubstrat 19a, 19b verwendet. Es handelt sich dabei um ein Keramiksubstrat mit einer Metall-, insbesondere einer Cr/Ni Galvanikstartschicht, auf welche eine Kupferschicht aufgebracht ist. Dies kann insbesondere mit einem Galvanisierungsverfahren erfolgen. So lassen sich auch mittel Galvanisierung Schichtdicken von über 10 µm, vorzugsweise bis zu 100 µm oder mehr erzeugen. Auch eine Ausgestaltung als direct printed copper Keramiksubstrat 19a, 19b ist denkbar. So können auch mittels eines Dickschicht-Druckverfahrens Kupferschichten von über 10 µm aufgebracht werden.

Mittels eines Sensors 17 ist im Betriebszustand bevorzugt die Intensität und/oder Wellenlänge von auf diesen einfallender elektromagnetischer Strahlung messbar.

Beispielsweise in Figur 9 ist die Anordnung der gedruckten Schaltungsplatine 19 und 19' erkennbar, bei welcher die jeweiligen LEDs 15 und Sensoren 17 jeweils dem vor diesen liegenden Fenster 8 zugewandt sind und die Schaltungsplatine 19, 19' mit deren Rückseite an einem Gehäusekörper 7.3 des Gehäuses 7.2 gehalten ist.

Bevorzugt ist jeweils eine LED 15 und ist jeweils ein Sensor 17 des jeweiligen Arrays 14, 16 hinter einem gemeinsamen Fenster 8 angeordnet.

Eine der LEDs 15 und/oder einer der Sensoren 17 kann jedoch auch jeweils hinter mehr als einem Fenstern 8 des Gehäuses 7.1, 7.2 angeordnet sein.

Der Controller 18 ist mittels einer Mehrleiterverbindung 20, welche sich, wie beispielsweise Figur 11 zu entnehmen durch das Innere der Durchführungs- und Halteeinrichtung 5 zu einem Verbinder 21 erstreckt, mit weiteren Versorgungs-und Steuerungseinrichtungen verbunden, welche jedoch um der Klarheit Willen in den Figuren nicht dargestellt sind.

Um der Klarheit Willen ist in Figur 11 nur eine Leitung der Mehrleiterverbindung 20 mit dem Verbinder 21 verbunden dargestellt.

Ebenfalls um der Klarheit Willen ist auch in Figur 12 jeweils nur eine Leitung der Mehrleiterverbindung 22 mit dem Verbinder 23 verbunden dargestellt.

Der Verbinder 23 des Gehäuses 7.1 des ersten Moduls ist zu dem Verbinder 24 des Gehäuses 7.2 des zweiten Moduls steckkompatibel, wodurch das Gehäuse 7.1 zumindest einen elektrischen Verbinder umfasst, welcher vorzugsweise anreihbar zu dem Verbinder 24 des weiteren Gehäuses 7.2 ausgebildet ist.

Der Verbinder 24 ist mittels einer weiteren Mehrleiterverbindung 25 mit der gedruckten Schaltungsplatine 19 der weiteren Quelle 13 zur Abgabe elektromagnetischer Strahlung, welche in dem Gehäuse 7.2 angeordnet ist, und insbesondere mit dem auf dieser gedruckten Schaltungsplatine angeordneten (jedoch in den Figuren nicht dargestellten) Controller 18 verbunden.

Die vorliegend offenbarten Mehrleiterverbindungen umfassen jeweils Zuleitungen und/oder Kontrollleitungen für die Vorrichtung 3 zur Abgabe elektromagnetischer Strahlung und zumindest eine von diesen ist durch zumindest eine Durchführung 5a geführt.

Hierdurch ist jedoch sowohl das Gehäuse 7.1 als auch das Gehäuse 7.2 modular anreihbar ausgebildet und können mit derartigen Modulen durch deren Anreihung vielfältigen Gegebenheiten jeweiliger und insbesondere bereits existierender Photobioreaktoren entsprochen werden.

Die Gehäuse 7.1 und 7.2 können insbesondere auch Befestigungsmittel zur modularen Anreihung umfassen, wie beispielsweise Passstifte eines ersten Gehäuses, welche in zugeordnete Passnuten oder passgenaue Sackloch-Bohrungen des jeweils zweiten Gehäuses eingreifen können.

Beispielhaft ist in Figur 12 lediglich der Passstift 26 des Gehäuses 7.2 des zweiten Moduls dargestellt, welcher in eine zugeordnete Bohrung 27 des Gehäuses 7.1 des ersten Moduls eingreift.

Nachfolgend wird wiederum auf Figur 14 Bezug genommen, welche sowohl die Mehrleiterverbindung 20, die mit dem Verbinder 21 verbunden ist, als auch die Mehrleiterverbindung 22 zeigt, welche mit dem Verbinder 23 verbunden ist. Die Mehrleiterverbindung 20, 22 sowie auch die Mehrleiterverbindung 25, welche den Verbinder 24 mit der gedruckten Schaltungsplatine 19 sowie deren Kontroller 18 verbindet, können sowohl Signale eines Kommunikationsbusses als auch jeweilige Versorgungsleitungen, insbesondere für die Kontroller 18 als auch die LEDs 15 und soweit nötig auch für Sensoren 17 umfassen.

Ein bevorzugter Kommunikationsbus umfasst beispielsweise den Datenaustausch über OPC XML und umfasst JAVAprogrammierbare Clients. Ein derartiger Kommunikationsbus ist mit dessen Clients beispielsweise beschrieben in SIMOTION, Beschreibung und Beispiel zum Datenaustausch über OPC XML Schnittstelle, Version 1.0 Ausgabe 07/2007 publiziert durch die Siemens AG.

Mittels des Kontrollers 18 der gedruckten Schaltungsplatine 19 ist auch der Stellmotor 27 des ersten Gehäuses 7.1 ansteuerbar. Der Stellmotor 27 ist stationär mit dem Gehäuse 7.1 verbunden, welches jedoch dem in Figur 11 dargestellten Pfeil entsprechend verdrehbar an der Durchführungs- und Halteeinrichtung 4 gehalten ist.

Mittels des von dem Stellmotor 27 angetriebenen Zahnrads 28, welches in kraftschlüssiger Verbindung mit dem mit der Durchführungs- und Halteeinrichtung fest verbundenen Zahnrad 29 kämmt, kann durch Rotation des Zahnrads 28, die Vorrichtung zur Abgabe elektromagnetischer Strahlung 3 in Richtung des in Figur 11 dargestellten Pfeils unter Steuerung des Kontrollers 18 definiert um deren Längsachse verschwenkt werden. Durch Änderung der Drehrichtung des Zahnrads 28 kann auch eine Verschwenkung entgegen der Richtung des in Figur 11 dargestellten Pfeils vorgenommen werden.

Durch diese Verschwenkung kann auch eine Justierung der nachfolgend noch detaillierter beschriebenen Messtrecken Mod und Moi erfolgen. Diese Justierung kann manuell gesteuert oder durch eine Prozesstezerungseinrichtung, welche mit den Controllern 18 kommuniziert, erfolgen.

Um die hermetische Dichtigkeit des Gehäuses 7.1 sicherzustellen weist sowohl die Durchführungs- und Halteeinrichtung 4 ein keramisches Dichtelement 30, als auch das Gehäuse 7.1 ein keramische Dichtelemente 31 auf.

Auch die Verbinder 21, 22 und 23 weisen jeweils zumindest ein keramisches Dichtelement 32, 33, 34 auf und stellen die Hermetizität des Gehäuses 7.1 und 7.2 auch hierdurch sicher. Darüber hinaus sind die keramischen Dichtelemente gegenüber Temperaturen, wie diese bei der Sterilisation verwendet werden, stabil, insbesondere bis über Temperaturen von 150° hinaus und sind auch beständig gegenüber chemischen Reinigungsmitteln, wie diese herkömmlich auch zur Reinigung von Photobioreaktoren oder Bioreaktoren verwendet wurden.

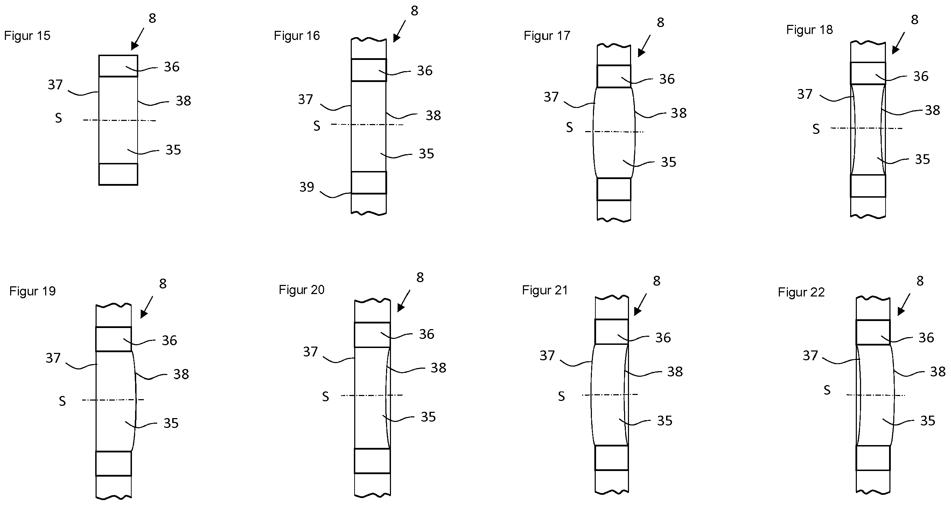

Bei den vorliegend bevorzugten Ausführungsformen bildet ein Fenster 8 oder bilden mehrere Fenster 8 jeweils eine Einglasung für ein transparentes Element 35, vorzugsweise eine GTMS-Druckeinglasung aus, wie dieses nachfolgend unter Bezugnahme auf Figur 15 beschrieben wird.

Figur 15 zeigt eine entlang der Symmetrieachse S verlaufende schematische Schnittdarstellung eines hier beschriebenen Fensters 8 zur Erläuterung von dessen Herstellungsverfahren.

Das in Figur 15 dargestellte Fenster 8 umfasst einen aus Stahl bestehenden, ring- oder zylinderförmigen Grundkörper 36, welcher das transparente Element 35 seitlich umgibt und auf dieses eine Druckkraft ausübt, die eine dauerhaft hermetische und für die Zwecke der vorliegenden Erfindung ausreichend druck- sowie temperaturfeste Verbindung zwischen dem transparenten Element 35 und dem Grundkörper 36 sicherstellt.

Das transparente Element 35 des jeweiligen Fensters 8 umfasst jeweils Glas oder besteht aus Glas.

Das Glas des transparenten Elements 35 der Fenster 8 kann beispielsweise jeweils ein Quarzglas oder auch ein Borosilikatglas umfassen oder aus diesem bestehen.

Bei seiner Herstellung wird das vorzugsweise entweder in etwa bereits seiner endgültigen Form entsprechende Element 35 innerhalb des Grundkörpers 36 angeordnet und mit diesem zusammen so lange erwärmt, bis das Glas des transparenten Elements 35 dessen Glastemperatur Tg oder dessen Halbkugeltemperatur überschritten hat und beginnt, am Grundkörper 36 anzuschmelzen.

Nach erfolgtem Anschmelzen wird dann der Verbund aus transparentem Element 35 und Grundkörper 36 auf Raumtemperatur abgekühlt, wodurch jeweils ein Fenster 8 mit einem im Wesentlichen scheibenförmigen transparentem Element 35 ausgebildet wird.

Dieser vorstehend beschriebene Vorgang, bei welchem das Glas des transparenten Elements 35 mit dem Grundkörper 36 verbunden wird, wird wie dem Fachmann bekannt ist, auch als anglasen und die dabei entstandene Verbindung des transparenten Elements 35 mit dem Grundkörper 36 als Einglasung bezeichnet.

Da der thermische Ausdehnungskoeffizient des Edelstahls des Grundkörpers 36 größer als der des Glases des transparenten Elements 35 ist, übt dieser, sobald das Glas des transparenten Elements sich zu verfestigen beginnt, eine mit weiter abnehmender Temperatur ansteigende Druckspannung auf das Glas des transparenten Elements 35 aus.

Der Grundkörper 36 hält das transparente Element 35 dann, nach erfolgter Abkühlung, mit dieser quasi eingefrorenen Druckspannung dauerbetriebsfest hermetisch dicht und temperaturstabil. Eine derartige Verbindung des transparenten Elements 35 mit dem Grundkörper 36 wird, wie dem Fachmann bekannt, auch als Druckeinglasung bezeichnet.

Eine derartige Druckverglasung wird im Rahmen der vorliegenden Offenbarung auch als Glas-Metall-Verbindung oder Glass-to-Metal-Seal, GTMS, bezeichnet, falls der Grundkörper 36 aus Metall besteht.

Bei einer derartigen Glass-to-Metall-Verbindung übt das Metall über den gesamten Betriebstemperaturbereich Druckkräfte auf das Glas aus, insbesondere auch bei Temperaturen bis zumindest 120° C, vorzugsweise sogar 140° Celsius, welche eine Druckspannung zwischen dem Metall und dem Glas ausbilden und dazu beitragen, dass die Glass-Metall-Verbindung dauerhaft betriebssicher sowohl fluidals auch hermetisch dicht ist.

Ferner treten bei derartigen Glas-Metall-Verbindungen in der Regel keine Spalte auf, wie diese bei Verwendung von herkömmlichen Dichtmitteln, beispielsweise von O-Ringen auftreten können und Raum für Verunreinigungen bieten, welche häufig nur schwer entfernbar sind.

Vorteilhaft ist hierbei ein Unterschied der thermischen Ausdehnungskoeffizienten, welcher die Druckspannung zwischen dem Glas des transparenten Elements 35 und dem Metall des ring- oder zylinderförmiger Grundkörper 36 des Fensters 8 zumindest über den Betriebstemperaturbereich zuverlässig aufrechterhält.

Dieser Unterschied des Ausdehnungskoeffizienten CTE_{M} des Metalls und dem Ausdehnungskoeffizienten des Glases CTE_{G} des transparenten Elements 3 kann beispielsweise kleiner als 80 * 10⁻⁶ /K, bevorzugt kleiner als 30 * 10⁻⁶ /K oder besonders bevorzugt auch kleiner als 20 * 10⁻⁶ /K betragen. Hierbei soll der thermische Ausdehnungskoeffizient des Metalls CTE_{M} jeweils größer als der thermische Ausdehnungskoeffizient des Glases CTE_{G} sein. In allen Fällen soll dieser Unterschied vorzugsweise jedoch mindestens 1 mal 10⁻⁶ /K betragen.

Quarzglas weist beispielsweise ein CTE_{G} von 0, 6 * 10⁻⁶ /K auf und kann beispielsweise mit Edelstählen mit einem CTE_{M} von 17-18 *10⁻⁶ /K kombiniert werden.

Wenn der Gehäusekörper 7.3 einen hochtemperaturbeständigen Kunststoff, insbesondere einen thermoplastischen Kunststoff wie einen Polyaryletherketon, insbesondere einen Polyetheretherketon, PEEK, umfasst oder aus diesem besteht, muss dieser Gehäusekörper 7.3 nicht unbedingt vollständig hermetisch sein, wie dieses im Rahmen der vorliegenden Offenbarung beschrieben wurde, sondern können dennoch durchaus wertvolle Betriebs- und Einsatzzeiten erreicht werden.

Es kann dann beispielsweise der Gehäusekörper 7.3 eine vorzugsweise runde Durchgangsöffnung aufweisen, welche etwa vom Verhältnis von deren Durchmessern her 1/10 kleiner als der Außendurchmesser des transparenten Elements 35 ist. Wenn der Gehäusekörper 7.3 auf eine Temperatur von etwa 200°C erhitzt wird, kann dann das transparente Element 35 in diese Durchgangsöffnung eingesetzt werden und es entsteht bei Abkühlung eine wie vorstehend beschriebene Druckspannung, beispielsweise von etwa 38 MPa, welche noch deutlich unter der Streckgrenze von 110 MPa von PEEK liegt.

In diesem dauerbetriebsfest hermetisch dichten und temperaturstabilen Zustand kann das Glas des transparenten Elements 35 entweder, vorzugsweise nach Prüfung der jeweiligen seitlichen Oberfläche entweder direkt verwendet oder weiteren Oberflächen-bearbeitenden Verfahren, wie beispielsweise Polieren oder formgebenden Schleifen zugeführt werden.

Auf diese Weise kann das jeweilige transparente Element 35 planparallele seitliche Hauptoberflächen 37 und 38 ausbilden oder kann das transparente Element 35 auch plankonvex, plankonkav, bikonvex, bikonkav, konvexkonkav oder konkavkonvex geformt werden, wie dieses den Schnittdarstellungen der Figuren 16 bis 22 zu entnehmen ist, bei welchen angenommen wird, dass sich die LEDs 15 jeweils links von dem transparenten Element 35 befinden.

Bei geringeren optischen Anforderungen an die Oberflächenqualität, insbesondere für beleuchtende Strahlengänge oder auch zur Messung verwendete Strahlengänge, kann das transparente Element 35 auch während des Herstellungsverfahrens in einer entsprechenden Negativ-Form gehalten sein, welche im Wesentlichen bereits dessen finaler Form entspricht.

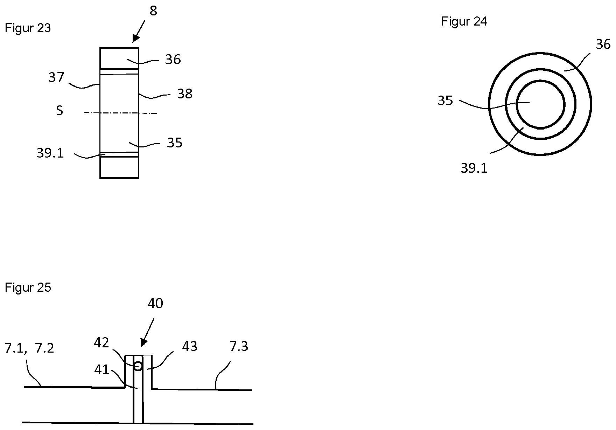

Wenn das transparente Element 35 des Fensters 8, wie es beispielsweise in den Figuren 23 und 24 gezeigt ist, aus Quarzglas besteht oder Quarzglas umfasst, kann anstelle der Laserschweißnaht 39 auch ein weiteres Glas 39.1 oder Glaslot 39.1 verwendet werden, um das Quarzglas hermetisch dicht mit dem ring- oder zylinderförmiger Grundkörper 36 des Fensters 8 fluid- und hermetisch dicht zu verbinden.

Das hierbei verwendete Glaslot ist vorzugsweise ein schwermetallfreies, insbesondere bleifreies Glaslot.

Der Photobioreaktor 1 sowie Vorrichtung zur Abgabe elektromagnetischer Strahlung 3 ist jeweils einzeln eigenständig oder es sind der Photobioreaktor 1 sowie Vorrichtung zur Abgabe elektromagnetischer Strahlung 3 auch zusammen autoklavierbar. Dies bedeutet, dass insbesondere die Vorrichtung zur Abgabe elektromagnetischer Strahlung 3 mit deren hier offenbarten Fenstern 8 hermetisch dicht ist und dabei einer Behandlung mit Sattdampf einer Temperatur von 121° C standhält, ohne dass der Sattdampf oder durch diesen gebildete Fluide in die Vorrichtung 3 eintreten können.

Autoklavierbar wird im Rahmen dieser Offenbarung auch als autoklavierbar im Sinne der DIN EN ISO 14937; EN ISO 17665, welche für Medizinprodukte gültig ist, verstanden.

Hierdurch wird auch das dem Fachmann bekannte und vorteilhafte Steaming-in-Place ermöglicht.

Vorteilhaft ist es auch, wenn hierbei innerhalb des Gehäuses 7, insbesondere des Gehäuses 7.1 und 7.1 der Module der Vorrichtung zur Abgabe elektromagnetischer Strahlung 3 ein gegenüber dem Äußeren des Gehäuses 7 der Vorrichtung zur Abgabe elektromagnetischer Strahlung 3 ein verminderter Druck herrscht.

Hierdurch wird nicht nur die Wärmeleitung innerhalb des Gehäuses 7, 7.1, 7.2 reduziert, sondern auch Korrosion der innerhalb des Gehäuses 7, 7.1, 7.2 angeordneten Baugruppen vermindert.

Figur 25 zeigt einen Querschnitt durch einen Teil des Gehäuse 7.1 des oberen oder ersten Moduls der Vorrichtung 3 zur Abgabe elektromagnetischer Strahlung oder auch des Gehäuses 7.2 des unteren oder zweiten Moduls der Vorrichtung zur Abgabe elektromagnetischer Strahlung 3, welche beide einen Evakuierungsport 40 aufweisen können.

Der Evakuierungsport 40 umfasst eine Durchgangsöffnung 41 durch eine Wand des Gehäuse 7.1 des oberen oder ersten Moduls der Vorrichtung 3 zur Abgabe elektromagnetischer Strahlung oder auch des Gehäuse 7.2 des unteren oder zweiten Moduls der Vorrichtung zur Abgabe elektromagnetischer Strahlung 3.

Der Evakuierungsport 40 weist in der Darstellung der Figur 25 eine in die Durchgangsöffnung 41 eingepresste Edelstahlkugel 42 auf, welche diese fluid- und hermetisch dicht abschließt.

Diese Edelstahlkugel 42 kann beispielsweise in die Durchgangsöffnung 41 eingepresst werden, nachdem beispielsweise innerhalb des Gehäuses 7.1, 7.2 der Vorrichtung zur Abgabe elektromagnetischer Strahlung 3 ein gegenüber dem Äußeren des Gehäuses verminderter Druck, beispielsweise durch deren Evakuierung hergestellt wurde.

Alternativ zur Verwendung der Edelstahlkugel 42 kann auch ein röhrenförmig hervorstehender Abschnitt 43 des Evakuierungsports 40 nach der Evakuierung verkrimpt, verlötet oder verschweisst werden, um eine fluid- und hermetisch dichten Verschluss bereit zu stellen.

Die Hauptoberflächen 37, 38 können eine Beschichtung aufweisen, welche wellenlängenselektiv ist und derart ein optisches Bandpass- oder Kantenfilter ausbilden. Mittels eines derartigen optischen Filters können mit der Vorrichtung 3 definierte Wellenlängen in den Behälter 2 eingestrahlt und identische oder von diesen abweichende Wellenlängen gemessen, insbesondere sensorisch erfasst werden. Diese Beschichtung kann nur auf einer oder auch auf beiden Hauptoberflächen vorhanden sein.

Ohne eine derartige, insbesondere ohne jegliche Beschichtung weist das transparente Element 35 zumindest eines Fensters 8 in einem Spektralbereich mit einer Wellenlänge von 250 bis 2000 nm eine Transmission auf, die höher ist als 80%, besonders bevorzugt höher als 90 %.

Bevorzugt wird der Grundkörper 36 der Fenster 8 direkt, insbesondere mittels Laser-Schweißen, jeweils hermetisch dicht mit dem Gehäusekörper 7.3 des jeweiligen Gehäuses 7, 7.1, 7.2 der Vorrichtung 3 zur Abgabe elektromagnetischer Strahlung verbunden, sodass hierdurch das Gehäuse 7, 7.1, 7.2 hermetisch dicht ausgebildet ist.

Als hermetisch dicht oder auch fluiddicht wird im Sinne der vorliegenden Offenbarung ein Gegenstand, beispielsweise die Vorrichtung 3 zur Abgabe elektromagnetischer Strahlung dann angesehen werden, wenn diese bei Raumtemperatur eine Leckrate von weniger als 1x10-3 mbar * l/sec bei einer Befüllung mit He und 1 bar Druckdifferenz aufweisen.

Beispielhaft ist die hierbei entstehende Laserschweißnaht in Figur 16 mit dem Bezugszeichen 39 dargestellt.

Wird nun Licht von einer der LEDs 15 ausgestrahlt, tritt dieses jeweils durch das vor dieser LED 15 angeordnete Fenster 8 und kommt es zu der in Figur 8 dargestellten Lichtverteilung.

Es bildet sich jeweils ein Lichtkegel mit einem Öffnungswinkel α vor einer sich in Längsrichtung erstreckende Seitenwand 7.5 des Gehäuses 7, 7.1, 7.2, welche jeweils eine Zeile von Fenstern 8 sowie die in Figur 14 dargestellte Quelle 13 zur Abgabe elektromagnetischer Strahlung hinter diesen Fenstern angeordnet aufweist.

Ferner bildet sich jeweils ein Lichtkegel mit einem Öffnungswinkel β vor einer sich in Längsrichtung erstreckende Seitenwand 7.6 des Gehäuses 7, 7.1, 7.2, welche jeweils zwei Zeilen von Fenstern 8 sowie die in Figur 13 dargestellte Quelle 13 zur Abgabe elektromagnetischer Strahlung hinter diesen Fenstern 8 angeordnet aufweist.

Diese Lichtkegel mit den Öffnungswinkeln α und β umfassen jeweils mehr als 95 % der Strahlungsintensität der von der jeweiligen LED 15 abgestrahlten elektromagnetischen Strahlung und werden im Wesentlichen in deren räumlicher Ausprägung jeweils durch die LED 15 sowie durch das dieser vor dieser angeordnete transparente optische Element 35 definiert.

Wie aus Figur 8 zu erkennen ist, wird hierdurch nahezu der gesamte Innenraum des Behälters 2 ausgeleuchtet.

Durch entsprechende Gestaltung der Hauptoberflächen 37 und 38 kann zusammen mit der Abstrahlcharakteristik der jeweiligen LED eine sehr homogene Abstrahlung erreicht werden, bei welcher innerhalb der Lichtkegel mit den Öffnungswinkeln α und β die Intensität der abgegebenen elektromagnetischen Strahlung nicht unter einen vordefinierten Minimalwert von etwa 30 % der Maximalintensität absinkt, falls der Behälter 2 leer ist und Verluste durch Streuung und Absorption sowie der Einfluss von Reflektion zu vernachlässigen ist.

Ferner definieren die LEDs 15 einer ersten, und in Figur 7 und 8 lediglich beispielhaft dargestellten Vorrichtung 3.1 zur Abgabe elektromagnetischer Strahlung zusammen mit den Sensoren 17 einer zweiten Vorrichtung 3.2 zur Abgabe elektromagnetischer Strahlung eine definierte Messeinrichtung zur Erfassung beispielsweise von Streu- und Absorptionsverlusten, welche in der durch die beiden Vorrichtungen 3.1 und 3.2 gebildeten Messtrecke Mod aufgetreten sind. Hierdurch können einer Trübungskammer entsprechende Messungen jeweils in situ vorgenommen werden.

Ferner ist diese Messung nicht auf nur zwei Vorrichtungen zur Abgabe elektromagnetischer Strahlung beschränkt, sondern können, wie beispielhaft in Figur 7 dargestellt ist auch entsprechende Messungen zwischen einer Vielzahl weiterer LEDs 15 und Sensoren 17 vorgenommen werden, wie dieses in Figur 7 beispielhaft durch weitere Messtrecken Mod und Moi dargestellt ist.

Neben der Messstrecke mit einer direkten optischen Verbindung, wie diese in Figur 8 mittels der Messtrecke Mod dargestellt ist, und bei welcher der bei dieser Messtrecke Mod messende Sensor 17 jeweils innerhalb des Lichtkegels mit dem Öffnungswinkel α oder β liegt, somit von der zu dieser Messtrecke Mod gehörenden LED 17 direkt angestrahlt würde, falls kein Fluid 9 im Behälter 2 wäre, existieren auch Messtrecken Moi mit einer quasi indirekten Beleuchtung des dieser Messtrecke zugeordneten optischen Sensors 17, welche ebenfalls nur beispielhaft in Figur 7 dargestellt sind.

Bei diesen Messtrecken Moi liegt der dieser Messtrecke zugeordnete Sensor 17 außerhalb des Lichtkegels mit dem Öffnungswinkel α oder β der dieser Messtrecke zugeordneten Leuchtdiode 15, kann aber dennoch auf dieser jeweiligen Messtrecke auftretendes Streulicht erfassen, welches durch das Fluid 9 mit dem darin befindlichen biologischen Material erzeugt wird.

Da die von den LEDs 15 ausgesendete elektromagnetische Strahlung auch in kurzen Pulsen abgeben werden kann, kann für eine definierte Zeit der nachfolgende Messzyklus durchgeführt werden.

Eine oder eine definierte Anzahl erster Leuchtdioden 15 gibt für ein kurzes Zeitintervall, beispielsweise von 1 bis 10 µs elektromagnetische Strahlung einer definierten Intensität ab. Während dieses Zeitintervalls kann ein Sensor 17 oder eine Vielzahl von Sensoren 17 die dabei empfangene Intensität der elektromagnetischen Strahlung messen.

Danach kann eine zweite Leuchtdiode für ein kurzes Zeitintervall, beispielsweise von 1 bis 10 µs elektromagnetische Strahlung einer definierten Intensität abgeben. Auch während dieses Zeitintervalls kann ein Sensor 17 oder eine Vielzahl von Sensoren 17 die dabei empfangene Intensität der elektromagnetischen Strahlung messen.

Dieser Vorgang kann fortgesetzt werden, bis der Behälter 2 in seinem Inneren mit der gewünschten örtlichen und zeitlichen Auflösung vollständig, insbesondere auch in seinem dynamischen oder auch statischen Verhalten erfasst ist.

Hierdurch wird eine sowohl jeweils im Zeitverhalten optimierte sensorische Erfassung und eine hierauf basierende sehr zeitnahe Optimierung der Prozesse innerhalb des Behälters 2 des Photobioreaktors 1 ermöglicht als auch eine örtlich aufgelöste sensorische Erfassung bereitgestellt und hierdurch eine sich über das gesamte erfasste Volumen innerhalb des Behälters 2 des Photobioreaktors erstreckende Optimierung, insbesondere gesteuerte Optimierung der Prozesse innerhalb des Behälters 2 des Photobioreaktors 1 verwirklicht.

Die entsprechende Ansteuerung der LEDs 15 sowie Auswertung der von den Sensoren 17 erfassten sensorischen Größen erfolgt jeweils mit den Controllern 18 und kann mittels der Mehrleiterverbindungen an eine zum Behälter 2 externe Prozessteuerungs- und zugeordnete Auswerteeinrichtungen kommuniziert und von dieser gesteuert werden.

Bei einer weiteren bevorzugten Ausführungsform wird ein N*M-Array von LEDs 15 und Sensoren 17 mit jeweils davor angeordneten Fenstern 8, verwendet, wobei N und M jeweils bevorzugt in einem Größenbereich von 2 bis 125 liegen kann.

Ferner kann bei Aussendung ebener Welle, wenn beispielsweise die LED 15 eine Laserdiode ist und ein planparalleles transparentes Element mit einem Öffnungswinkel von nahezu 0° verwendet wird, mit einem zweiten zugehörigen Sensorarray eine zeilenweise Messung im Inneren des Behälters 2 vorgenommen werden, welche nahezu das gesamte innere Volumen des Behälters 2 erfassen kann.

Hierdurch wird eine besonders vorteilhafte Regulierung der reellen tatsächlichen Intensität der elektromagnetischen Strahlung im Fluid ermöglicht. Ferner ist eine dynamische Anpassung an die Sollintensität möglich. Auch eine Kompensation der variierenden Ausleuchtungstiefe, welche durch die wechselnde/steigende Zelldichte während der Kultivierung bedingt sein kann, wird in Echtzeit oder zumindest mit nur geringem zeitlichen Versatz ermöglicht.

Ebenso ist eine Kompensation des Substratverbrauchs und/oder durch Produktbildung bedingte Adsorptionsänderungen im Fluid sowohl jeweils örtlich als auch zeitlich aufgelöst möglich.

Der Photobioreaktor 1 erlaubt somit mittels den hier beschriebenen Vorrichtungen 3, 3.1 und 3.2 eine sowohl zeitlich als auch örtlich aufgelöste in situ Messung der optischen Absorption, der Streuung sowie von fluoreszenten Signalen des biologischen Materials 10 und Nutzung dieser Messsignale zur Optimierung der erwünschten Stoffwechselvorgänge des biologischen Materials 10.

Basierend auf diesen Messsignalen kann auch eine wesentlich effizientere Zufuhr von Nährlösung zeitlich und örtlich aufgelöst erfolgen, wenn beispielsweise der Photobioreaktor über entsprechende weitere, jedoch in den Figuren nicht dargestellte Zuführungseinrichtungen verfügt.

Ferner kann entlang jeder der vorstehend beschriebenen Messtrecken eine in-situ-Wirksamkeitsmessung als Wellenlängeneindringtiefenmessung als Indikator für Wachstum des biologischen Materials 10 vorgenommen werden.

Der Photobioreaktor 1 kann in dem Fachmann bekannter Weise zusätzlich noch Agitatoren zur Bewegung des fluiden Medium umfassen, welche in den Figuren jedoch um der Klarheit Willen nicht dargestellt sind. Ferner kann der Photobioreaktor Zuführungen, Temperierungseinrichtungen und strömungsbeeinflussende Einrichtungen enthalten, welche dem Fachmann zwar bekannt aber ebenfalls in den Figuren nicht dargestellt sind.

Die hier beschriebenen Module der Vorrichtung zur Abgabe elektromagnetischer Strahlung werden bevorzugt im günstigen Bereich der Strömungsverteilung des Bioreaktors angebracht, insbesondere bei Photobioreaktoren mit Rührwerken. Gegenüber Röhrenreaktoren ergibt sich im statistischen Mittel dann eine homogenere Verteilung der Lichtquanten auf die Zellen.

Mit den hier beschriebenen Vorrichtungen kann insbesondere auch ein UpScale und Transfer vom Labor- in den Produktionsmassstab von Kultivierungen von phototrophen Mikroorganismen unter sterilen Bedingungen ermöglicht werden.

Die Erfindung umfasst jedoch nicht nur Vorrichtungen, sondern auch Verfahren.

Eines dieser Verfahren ist ein Verfahren zur Vermehrung oder Kultivierung biologischen Materials vorzugsweise zur Herstellung von Pharmazeutika, insbesondere Biopharmazeutika, welches umfasst: das Einbringen biologischen Materials oder einer Vorstufe biologischen Materials in einen Photobioreaktor, wie dieser hier offenbart wird, das Einwirkenlassen elektromagnetischer Strahlung, welche von einer Vorrichtung zur Abgabe elektromagnetischer Strahlung, wie diese vorliegend offenbart wird, emittiert wurde.

Ein weiteres Verfahren umfasst ein Verfahren zum Präparieren von biologischem Material und/oder Herstellen von Pharmazeutika, insbesondere Biopharmazeutika, welches das Bereitstellen eines Photobioreaktors 1, der mit zumindest einer Vorrichtung zur Abgabe elektromagnetischer Strahlung, wie diese vorliegend offenbart wird, bestückt wird.

Bei diesen Verfahren weist der Photobioreaktor 1 zumindest eine Durchführung 5a auf, insbesondere zumindest einen Standard-Sparger-Port 5, wobei Zuleitungen und/oder Kontrollleitungen, insbesondere eine Mehrleiterverbindung 20 für die Vorrichtung 3 zur Abgabe elektromagnetischer Strahlung durch die zumindest eine Durchführung 5a geführt werden. Hierdurch kann beispielsweise ein Controller 18, welcher bevorzugt auf der gedruckten Schaltungsplatine 19, 19' einer Quelle 13 zur Abgabe elektromagnetischer Strahlung angeordnet ist, mittels der Mehrleiterverbindung 20 oder auch der Mehrleiterverbindungen 22, 25 mit einer zum Behälter 2 externe Prozessteuerungs- und zugeordnete Auswerteeinrichtungen kommunizieren und auch von dieser gesteuert werden.

Die gedruckte Schaltungsplatine 19 kann hierbei aus einem Material bestehen, welches eine Temperaturfestigkeit von beispielsweise 380° C aufweist, welches insbesondere für die Zyklenfestigkeit beim Autoklavieren vorteilhaft ist.

Bei den hier offenbarten Verfahren weist der Photobioreaktor 1 in seinem Innern, insbesondere dem Inneren des Behälters 2 zur Aufnahme biologischen Materials bevorzugt zumindest eine Montageeinrichtung 6 zum Fixieren von Prall- oder Verwirbelungsblechen auf und wird die zumindest eine Vorrichtung zur Abgabe elektromagnetischer Strahlung 3 mit zumindest dieser einen Montageeinrichtung 6 verbunden, wobei bevorzugt die Vorrichtung 3 zur Abgabe elektromagnetischer Strahlung durch die zumindest eine Durchführung 5a und die zumindest eine Montageeinrichtung 6 im Innern des Photobioreaktors 1, insbesondere dem Inneren des Behälters 2zur Aufnahme biologischen Materials fixiert werden.

Generell und insbesondere bei Durchführung der hier offenbarten Verfahren kann der Photobioreaktor 1 auch nach Bestückung mit der Vorrichtung 3 zur Abgabe elektromagnetischer Strahlung sterilisiert wird.

Bei diesen Verfahren weist zumindest eine Vorrichtung 3 zur Abgabe elektromagnetischer Strahlung vorteilhaft zumindest einen Sensor 17 zur Messung elektromagnetischer Strahlung auf, und wird im Betriebszustand die Strahlungsintensität und/oder Wellenlänge der elektromagnetischen Strahlung im Innern des Behälters 2 Photobioreaktors 1 gemessen, und wird im Betriebszustand die Strahlungsintensität und/oder Wellenlänge der elektromagnetischen Strahlung im Innern des Behälters 2 Photobioreaktors 1 bevorzugt zeitlich und örtlich aufgelöst gemessen.

Ferner kann bei den hier beschriebenen Verfahren für einen definierten Zeitraum elektromagnetische Strahlung einer definierten Wellenlänge, bevorzugt 250 nm in den Photobioreaktor eingestrahlt werden und nach dieser Einstrahlung breitbandig eine Strahlungsintensität und/oder Wellenlänge der elektromagnetischen Strahlung im Innern des Behälters 2 Photobioreaktors 1 gemessen werden.

Darüber hinaus kann ebenfalls für einen definierten Zeitraum, bevorzugt während einer Produktionsphase elektromagnetische Strahlung einer definierten Wellenlänge, bevorzugt 250 nm in den Photobioreaktor eingestrahlt werden und nach dieser Einstrahlung elektromagnetische Strahlung einer definierten weiteren Wellenlänge, bevorzugt in einem Bereich von 620 bis 780 nm eingestrahlt werden.

Bei diesen Verfahren können vorteilhaft insbesondere auch durch Mutagenese veränderte photo- oder mixotrophe Mikroorganismen verwendet werden.

Die hier beschriebenen Verfahren können im Batch-Betrieb, im Fed-Batch-Betrieb mit definierter Feedrate, insbesondere örtlich und zeitlich aufgelöster sowie gesteuerter Feedrate durchgeführt werden und vorteilhaft dabei durch Einstrahlung geeigneter Wellenlängen, beispielsweise von 250 nm eine Inhibierung, insbesondere Substratinhibierung bewirkten, welche zu einer erhöhten Erzeugungsrate des biologischen Materials führt.

Ferner kann auch durch eine zeitlich und örtlich aufgelöste und vorzugsweise kontrolliert gesteuerte Nährlösungsvorlage, insbesondere Zuckervorlage und Nachfütterung eine Stimulation der Produktbildung bewirkt werden.

Die hier beschriebenen Verfahren können ebenfalls im Repeated-Batch-Betrieb eingesetzt werden.

Da die Optima der Wellenlängen stets stammspezifisch sind, kann diesem durch eine geeignete Auswahl der LEDs 15 oder Laserdioden 15 sowie der beschriebenen Beschichtung des transparenten Elements 35 Rechnung getragen werden.

Neben der Produktion ist die Erfindung auch für die Prozessentwicklung sehr vorteilhaft. Insbesondere für die letztgenannte kann eine flexible Anpassung der Wellenlängen nochmals weitere Vorteile mit sich bringen.

Vorteilhaft kann die Vorrichtung zur Abgabe elektromagnetischer Strahlung auch über einen oder mehrere Sensoren zur Messung des CO₂-Gehaltes innerhalb des Bioreaktors verfügen.

Um einen Photobioreaktor mit einer Vorrichtung zur Abgabe elektromagnetischer Strahlung sowie eine Vorrichtung zur Abgabe elektromagnetischer Strahlung für einen Photobioreaktor bereitzustellen, welche eine möglichst effiziente Nutzung des Photobioreaktors ermöglichen und bei welchen eine Kontamination des Inneren des Photobioreaktors vermindert wird, ist generell erfindungsgemäß vorgesehen, dass die Vorrichtung zur Abgabe elektromagnetischer Strahlung mit deren Gehäuse innerhalb des Behälters zur Aufnahme von biologischem Material angeordnet ist.

### Bezugszeichenliste

- 1: Photobioreaktor
- 2: Behälter zur Aufnahme von fluiden Medien, die biologisches Material enthalten, auch als Behälter zur Aufnahme von biologischem Material bezeichnet
- 3: Vorrichtung zur Abgabe elektromagnetischer Strahlung
- 4: Durchführungs- und Halteeinrichtung
- 5: Sparger-Port, insbesondere Standart-Sparger-Port, welcher jeweils eine Durchführung 5a ausbildet
- 6: Montageeinrichtung
- 7: Gehäuse der Vorrichtung zur Abgabe elektromagnetischer Strahlung
7.1 Gehäuse des oberen oder ersten Moduls der Vorrichtung zur Abgabe elektromagnetischer Strahlung
7.2 Gehäuse des unteren oder zweiten Moduls der Vorrichtung zur Abgabe elektromagnetischer Strahlung
7.3 Gehäusekörper des jeweiligen Gehäuses 7, 7.1, 7.2 der Vorrichtung 3 zur Abgabe elektromagnetischer Strahlung
7.4 Sich in Längsrichtung erstreckende Seitenwand des Gehäuses 7, 7.1, 7.2
7.5 Sich in Längsrichtung erstreckende Seitenwand des Gehäuses 7, 7.1, 7.2
7.6 Sich in Längsrichtung erstreckende Seitenwand des Gehäuses 7, 7.1, 7.2
- 8: Fenster
- 9: Fluide Medium, welches biologisches Material enthält
- 10: Biologisches Material
- 11: Oberfläche des fluiden Mediums
- 12: Minimaler Abstand des Gehäuses 7 oder des Gehäuse 7.1, 7.2 des jeweiligen Moduls der Vorrichtung 3 zur Abgabe elektromagnetischer Strahlung zur Wand, insbesondere zur Innenwand des Behälters 2
- 13: Quelle zur Abgabe elektromagnetischer Strahlung
- 14: Einzeiliger Array von LEDs 15
- 15: LED
- 16: Einzeiliger Array von Sensoren 17
- 17: Sensor zur Messung der Intensität oder Wellenlänge elektromagnetischer Strahlung
- 18: Controller
- 19: 19' Gedruckte Schaltungsplatine (PCB) der Ausführungsform der Figur 14 sowie der Ausführungsform der Figur 13 19a keramisches Substrat 19b keramisches Substrat
- 20: Mehrleiterverbindung
- 21: Verbinder
- 22: Mehrleiterverbindung
- 23: Verbinder des Gehäuses 7.1 des ersten Moduls
- 24: Verbinder des Gehäuses 7.2 des zweiten Moduls
- 25: Mehrleiterverbindung
- 26: Passstift des Gehäuses 7.2
- 27: Stellmotor
- 28: Antriebs-Zahnrad
- 29: Abtriebs-Zahnrad
- 30: Keramisches Dichtelement
- 31: Keramisches Dichtelement
- 32: Keramisches Dichtelement
- 33: Keramisches Dichtelement
- 34: Keramisches Dichtelement
- 35: Transparentes Element des Fensters 8
- 36: Ring- oder zylinderförmiger Grundkörper des Fensters 8
- 37: Seitliche Hauptoberfläche des transparenten Elements 35
- 38: Seitliche Hauptoberfläche des transparenten Elements 35
- 39: Laserschweißnaht
- 39.1: Glaslot
- 40: Evakuierungsport
- 41: Durchgangsöffnung
- 42: Edelstahlkugel
- 43: Hervorkragender röhrenförmiger Abschnitt

## Patentansprüche

1. Photobioreaktor umfassend
einen Behälter zur Aufnahme von fluiden Medien, die biologisches Material enthalten,
eine Vorrichtung zur Abgabe elektromagnetischer Strahlung, mit einem Gehäuse, in welchem eine Quelle zur Abgabe elektromagnetischer Strahlung angeordnet ist,
**dadurch gekennzeichnet, dass**
die Vorrichtung zur Abgabe elektromagnetischer Strahlung mit deren Gehäuse innerhalb des Behälters zur Aufnahme von biologischem Material angeordnet ist und ein oder mehrere Fenster aufweist, welche jeweils eine Einglasung ausbilden.

2. Photobioreaktor nach Anspruch 1, **gekennzeichnet durch** zumindest eine Durchführung, welche durch zumindest eine Wand des Photobioreaktors führt und
bei welchem die Vorrichtung zur Abgabe elektromagnetischer Strahlung an zumindest einer Durchführung gehalten ist.

3. Photobioreaktor nach einem der vorstehenden Ansprüche, **dadurch gekennzeichnet, dass** der Photobioreaktor in seinem Inneren, insbesondere dem Inneren des Behälters zur Aufnahme biologischen Materials zumindest eine Montageeinrichtung zum Fixieren, insbesondere von Prall- oder Verwirbelungsblechen aufweist und die zumindest eine Vorrichtung zur Abgabe elektromagnetischer Strahlung mit zumindest dieser einen Montageeinrichtung verbunden ist.

4. Photobioreaktor nach einem der vorstehenden Ansprüche, **dadurch gekennzeichnet, dass** der Photobioreaktor zusammen mit der Vorrichtung zur Abgabe elektromagnetischer Strahlung autoklavierbar ist, insbesondere während diese mit deren Gehäuse innerhalb des Behälters zur Aufnahme von biologischem Material angeordnet ist, autoklavierbar ist.

5. Vorrichtung zur Abgabe elektromagnetischer Strahlung für einen Photobioreaktor, insbesondere für einen Photobioreaktor nach einem der vorstehenden Ansprüche, umfassend ein Gehäuse, in welchem die Quelle zur Abgabe elektromagnetischer Strahlung angeordnet ist
und ein oder mehrere Fenster aufweist, welche jeweils eine Einglasung ausbilden und bei welcher vorzugsweise die Quelle zur Abgabe elektromagnetischer Strahlung LEDs, insbesondere einen Array von LEDs umfasst.

6. Vorrichtung zur Abgabe elektromagnetischer Strahlung nach Anspruch 5, wobei in dem Gehäuse zumindest ein Sensor zum Messen von elektromagnetischer Strahlung angeordnet ist, wobei mittels des Sensors im Betriebszustand bevorzugt die Intensität und/oder Wellenlänge von einfallender elektromagnetischer Strahlung messbar und/oder einstellbar, insbesondere geregelt einstellbar ist.

7. Vorrichtung zur Abgabe elektromagnetischer Strahlung nach Anspruch 5 oder 6,
**dadurch gekennzeichnet, dass** die LEDs und/oder der zumindest eine Sensor hinter dem einem oder den mehreren Fenstern des Gehäuses angeordnet sind.

8. Vorrichtung zur Abgabe elektromagnetischer Strahlung nach Anspruch 7, **dadurch gekennzeichnet, dass** das eine oder die mehreren Fenster jeweils eine GTMS-Druckeinglasung ausbilden
und/oder ein Grundkörper der Fenster direkt, insbesondere mittels Laser-Schweißen, jeweils hermetisch dicht mit dem Gehäusekörper verbunden und das Gehäuse hermetisch dicht ausgebildet ist.

9. Vorrichtung zur Abgabe elektromagnetischer Strahlung nach Anspruch 7 oder 8, **dadurch gekennzeichnet, dass** zumindest ein transparentes Element eines Fensters scheibenförmig ausgebildet ist und insbesondere planparallele Hauptoberflächen aufweist oder plankonvex, plankonkav, bikonvex, bikonkav, konvexkonkav oder konkavkonvex geformt ist.

10. Vorrichtung zur Abgabe elektromagnetischer Strahlung nach einem der Ansprüche 7, 8 oder 9, **dadurch gekennzeichnet, dass** das transparente Element des jeweiligen Fensters Glas umfasst oder aus Glas besteht
und das Glas des transparenten Elements der Fenster vorzugsweise Quarzglas oder Borosilikatglas umfasst.

11. Vorrichtung zur Abgabe elektromagnetischer Strahlung nach einem der Ansprüche von 7 bis 10, **dadurch gekennzeichnet, dass** das transparente Element zumindest eines Fensters in einem Spektralbereich mit einer Wellenlänge von 250 bis 2000 nm eine Transmission aufweist, die höher ist als 80%, besonders bevorzugt höher als 90 %.

12. Vorrichtung zur Abgabe elektromagnetischer Strahlung nach einem der Ansprüche von 5 bis 11 **dadurch gekennzeichnet, dass** das Gehäuse einen Gehäusekörper umfasst, welcher als Frästeil ausgebildet ist, und/oder der Gehäusekörper säulenförmig ausgebildet ist und zwei von dessen sich in Längsrichtung erstreckenden Seitenwänden einen 30° Winkel und zwei von dessen sich in Längsrichtung erstreckenden Seitenwänden einen 60° Winkel zueinander aufweisen, wobei vorzugsweise zumindest zwei der sich in Längsrichtung erstreckenden Seitenwände Fenster umfassen.

13. Vorrichtung zur Abgabe elektromagnetischer Strahlung nach einem der Ansprüche von 5 bis 12, **dadurch gekennzeichnet, dass** das Gehäuse zumindest einen elektrischen Verbinder umfasst, welcher vorzugsweise anreihbar mit einem Verbinder eines weiteren Gehäuses ausgebildet ist und/oder das Gehäuse modular anreihbar ausgebildet ist und insbesondere Befestigungsmittel zur modularen Anreihung umfasst.

14. Vorrichtung zur Abgabe elektromagnetischer Strahlung nach einem der Ansprüche von 5 bis 13, **dadurch gekennzeichnet, dass** innerhalb des Gehäuses der Vorrichtung zur Abgabe elektromagnetischer Strahlung ein gegenüber dem Äußeren des Gehäuses verminderter Druck herrscht und insbesondere das Gehäuse einen fluiddicht verschließbaren Evakuierungsport aufweist.

15. Verfahren zur Vermehrung oder Kultivierung biologischen Materials vorzugsweise zur Herstellung von Pharmazeutika, insbesondere Biopharmazeutika umfassend
das Einbringen biologischen Materials oder einer Vorstufe biologischen Materials in einen Photobioreaktor nach einem der Ansprüche von 1 bis 4, das Einwirkenlassen elektromagnetischer Strahlung, welche von einer Vorrichtung zur Abgabe elektromagnetischer Strahlung nach einem der Ansprüche von 5 bis 14 emittiert wurde.

16. Verfahren zum Präparieren von biologischem Material und/oder Herstellen von Pharmazeutika, insbesondere Biopharmazeutika, insbesondere gemäß einem Verfahren nach Anspruch 15, umfassend das Bereitstellen eines Photobioreaktors, der mit zumindest einer Vorrichtung zur Abgabe elektromagnetischer Strahlung nach einem der Ansprüche von 5 bis 14 bestückt wird und bei welchem für einen definierten Zeitraum elektromagnetische Strahlung einer definierten Wellenlänge, bevorzugt 250 nm in den Photobioreaktor eingestrahlt wird und nach dieser Einstrahlung breitbandig oder selektiv bei einer Wellenlänge, insbesondere bei 270 nm eine Strahlungsintensität und/oder Wellenlänge der elektromagnetischen Strahlung im Innern des Bioreaktors gemessen wird und/oder
bei welchem für einen definierten Zeitraum elektromagnetische Strahlung einer definierten Wellenlänge, von bevorzugt 250 nm in den Photobioreaktor eingestrahlt wird und nach dieser Einstrahlung elektromagnetische Strahlung einer definierten weiteren Wellenlänge, bevorzugt in einem Bereich von 620 bis 780 nm in den Photobioreaktor eingestrahlt wird.

17. Verfahren nach Anspruch 15 oder 16, bei welchem durch Mutagenese veränderte photo- oder mixotrophen Mikroorganismen, insbesondere auch Mikroalgen, Hefen und Bakterien, verwendet werden.

18. Verfahren nach einem der Ansprüche von 15 bis 17, bei welchem der Photobioreaktor mit dessen Behälter zur Aufnahme von fluiden Medien, die biologisches Material enthalten, und der Vorrichtung zur Abgabe elektromagnetischer Strahlung autoklaviert wird während die Vorrichtung zur Abgabe elektromagnetischer Strahlung mit deren Gehäuse innerhalb des Behälters zur Aufnahme von biologischem Material angeordnet ist.

## Claims

1. A photobioreactor, comprising
a container for holding fluid media containing biological material;
a device for emitting electromagnetic radiation, comprising a housing with a source for emitting electromagnetic radiation arranged therein;
**characterised in that**
the device for emitting electromagnetic radiation including the housing thereof is arranged inside the container for holding biological material, and has one or more windows, each of which forms a glass seal.

2. The photobioreactor according to claim 1, **characterised by** at least one feedthrough extending through at least one wall of the photobioreactor; and
wherein the device for emitting electromagnetic radiation is supported on at least one feedthrough.

3. The photobioreactor according to any one of the preceding claims, **characterised in that** the photobioreactor comprises, in its interior, in particular in the interior of the container for holding biological material, at least one mounting means for fixing in particular baffles or turbulence metal sheets, and wherein the at least one device for emitting electromagnetic radiation is coupled to at least this one mounting means.

4. The photobioreactor according to any one of the preceding claims, **characterised in that** the photobioreactor is autoclavable together with the device for emitting electromagnetic radiation, in particular while the latter including the housing thereof is arranged inside the container for holding biological material.

5. A device for emitting electromagnetic radiation for a photobioreactor, in particular for a photobioreactor according to any one of the preceding claims, comprising a housing with the source for emitting electromagnetic radiation arranged therein, and having one or more windows, each of which forms a glass seal;
and wherein the source for emitting electromagnetic radiation comprises LEDs, in particular an array of LEDs.

6. The device for emitting electromagnetic radiation according to claim 5, wherein at least one sensor for measuring electromagnetic radiation is arranged in the housing, wherein in the operating state the sensor can be used to measure and/or adjust preferably the intensity and/or wavelength of incident electromagnetic radiation, in particular in a controlled manner.

7. The device for emitting electromagnetic radiation according to any one of claims 5 or 6, **characterised in that** the LEDs and/or the at least one sensor are arranged behind the one or more windows of the housing.

8. The device for emitting electromagnetic radiation according to claim 7, **characterised in that** each of the one or more windows forms a GTMS compression glass seal;
and/or that a main body of the windows is directly joined to the housing body in a hermetically sealed manner, in particular by laser welding, and the housing is hermetically sealed.

9. The device for emitting electromagnetic radiation according to claims 7 or 8, **characterised in that** at least one transparent element of a window has a sheet-like shape and has in particular plane-parallel main surfaces, or has a plano-convex, plano-concave, biconvex, biconcave, convexo-concave, or concavo-convex shape.

10. The device for emitting electromagnetic radiation according to any one of claims 7, 8 or 9, **characterised in that** the transparent element of the respective window comprises glass or is made of glass; and that the glass of the transparent element of the windows preferably comprises fused silica or borosilicate glass.

11. The device for emitting electromagnetic radiation according to any one of claims 7 to 10, **characterised in that** the transparent element of at least one window exhibits a transmittance of greater than 80 %, most preferably greater than 90 %, in a spectral range of wavelengths between 250 and 2000 nm.

12. The device for emitting electromagnetic radiation according to any one of claims 5 to 11, **characterised in that** the housing comprises a housing body which is formed as a milled part; and/or that the housing body has a columnar shape with longitudinally extending side walls, wherein two of the longitudinally extending side walls thereof enclose an angle of 30°, and two of the longitudinally extending side walls thereof enclose an angle of 60°, wherein at least two of the longitudinally extending side walls have windows.

13. The device for emitting electromagnetic radiation according to any one of claims 5 to 12, **characterised in that** the housing comprises at least one electrical connector which is configured to be preferably serially connectable to a connector of a further housing, and/or that the housing is configured for being coupled serially in a modular manner and in particular comprises fastening means for modular serial coupling.

14. The device for emitting electromagnetic radiation according to any one of claims 5 to 13, **characterised in that** a pressure is prevailing within the housing of the device for emitting electromagnetic radiation, which pressure is reduced relative to the exterior of the housing, and wherein the housing in particular has an evacuation port that can be sealed fluid-tightly.

15. A method for propagation or cultivation of biological material, preferably for producing pharmaceuticals, in particular biopharmaceuticals, comprising
introducing of biological material or of a precursor of biological material into a photobioreactor according to any one of claims 1 to 4;
exposing to electromagnetic radiation emitted by a device for emitting electromagnetic radiation according to any one of claims 5 to 14.

16. A method for preparing biological material and/or for producing pharmaceuticals, in particular biopharmaceuticals, in particular according to a method according to claim 15, comprising the providing of a photobioreactor that is equipped with at least one device for emitting electromagnetic radiation according to any one of claims 5 to 14, and wherein electromagnetic radiation of a predefined wavelength, preferably 250 nm, is irradiated into the photobioreactor over a predefined duration, and wherein after this irradiation, a radiation intensity and/or a wavelength of the electromagnetic radiation is measured inside the bioreactor over a broad range of wavelengths or selectively at a particular wavelength, in particular at 270 nm; and/or
wherein electromagnetic radiation of a predefined wavelength, preferably 250 nm, is irradiated into the photobioreactor over a predefined duration, and wherein after this irradiation, electromagnetic radiation of a predefined further wavelength is irradiated into the photobioreactor, preferably in a range from 620 to 780 nm.

17. The method according to any one of claims 15 to 16, wherein photo- or mixotrophic microorganisms modified by mutagenesis are used, in particular microalgae, yeasts, and bacteria.

18. The method according to any one of claims 15 to 17, wherein the photobioreactor together with its container for holding fluid media containing biological material and the device for emitting electromagnetic radiation is autoclaved while the device for emitting electromagnetic radiation including the housing thereof is arranged inside the container for holding biological material.

## Revendications

1. Photobioréacteur comprenant
un récipient destiné à la reception de milieux fluides qui contiennent de la matière biologique,
un dispositif destiné à l'émission de rayonnement électromagnétique, comportant un boîtier dans lequel est disposée une source destinée à l'émission de rayonnement magnétique,
**caractérisé en ce que**
le dispositif destiné à l'émission de rayonnement électromagnétique est dispose avec son boîtier à l'intérieur du récipient destiné à la reception de matière biologique et comporte une ou plusieurs fenêtre(s) qui constituent chacune un vitrage.

2. Photobioréacteur selon la revendication 1, **caractérisé par** au moins une voie de passage qui traverse au moins une paroi du photobioréacteur et dans lequel le dispositif destiné à l'émission de rayonnement électromagnétique est maintenu au niveau d'au moins une voie de passage.

3. Photobioréacteur selon l'une quelconque des revendications précédentes, **caractérisé en ce que** le photobioréacteur comporte dans son intérieur, en particulier à l'intérieur du récipient destiné à la reception de matière biologique, au moins un dispositif de montage destiné à la fixation, en particulier de tôles de chicanes ou de tourbillonnement et ledit au moins un dispositif destiné à l'émission de rayonnement électromagnétique est relié à au moins ce dit un dispositif de montage.

4. Photobioréacteur selon l'une quelconque des revendications précédentes, **caractérisé en ce que** le photobioréacteur est autoclavable conjointement avec le dispositif destiné à l'émission de rayonnement électromagnétique, en particulier est autoclavable alors que ce dernier est disposé avec son boîtier à l'intérieur du récipient destiné ä la reception de matière biologique.

5. Dispositif destiné à l'émission de rayonnement électromagnétique pour un photobioréacteur, en particulier pour un photobioréacteur selon l'une quelconque des revendications précédentes, comprenant un boîtier, dans lequel est disposée la source destinée à l'émission de rayonnement electromagnétique, et comporte une ou plusieurs fenêtre(s) qui constituent chacune un vitrage, et dans lequel de preference la source destinée à l'émission de rayonnement électromagnétique comprend des LED, en particulier un groupement de LED.

6. Dispositif destiné à l'émission de rayonnement électromagnétique selon la revendication 5, dans lequel au moins un capteur destiné à la mesure de rayonnement électromagnétique est disposé dans le boîtier, dans l'état de fonctionnement de preference l'intensité et/ou la longueur d'onde de rayonnement électromagnétique incident étant mesurable(s) et/ou réglable(s), en particulier réglable(s) de façon commandée, au moyen du capteur.

7. Dispositif destiné à l'émission de rayonnement électromagnétique selon la revendication 5 ou 6,
**caractérisé en ce que** les LED et/ou ledit au moins un capteur sont disposés derrière ladite une ou lesdites plusieurs fenêtre(s) du boîtier.

8. Dispositif destiné à l'émission de rayonnement électromagnétique selon la revendication 7, **caractérisé en ce que** ladite une ou lesdites plusieurs fenêtre(s) constituent chacune un vitrage resistant à la pression GTMS
et/ou un corps de base des fenêtres est assemblé directement, en particulier par soudage laser, chaque fois de façon hermétiquement étanche avec le corps de boîtier et le boîtier est conçu hermétiquement étanche.

9. Dispositif destiné ä l'émission de rayonnement électromagnétique selon la revendication 7 ou 8, **caractérisé en ce qu'**au moins un élément transparent d'une fenêtre est configure en forme de disque et en particulier présente des surfaces principales planes et paralleles ou est en forme plano-convexe, plano-concave, biconvexe, biconcave, convexe-concave ou concave-convexe.

10. Dispositif destiné à l'émission de rayonnement électromagnétique selon l'une quelconque des revendications 7, 8 et 9, **caractérisé en ce que** l'élément transparent de la fenêtre respective comprend du verre ou est constitué de verre
et le verre de l'élément transparent de la fenêtre comprend de préférence du verre de quartz ou du verre de borosilicate.

11. Dispositif destiné à l'émission de rayonnement électromagnétique selon l'une quelconque des revendications 7 ä 10, **caractérisé en ce que** l'élément transparent d'au moins une fenêtre présente dans une region spectrale ayant une longueur d'onde de 250 ä 2 000 nm une transmission qui est supérieure à 80 %, de façon particulièrement préférée supérieure à 90 %.

12. Dispositif destiné à l'émission de rayonnement électromagnétique selon l'une quelconque des revendications 5 à 11, **caractérisé en ce que** le boîtier comprend un corps de boîtier qui est configuré sous forme de piece fraisée, et/ou le corps de boîtier est configuré sous forme de colonne et deux de ses parois laterales qui s'étendent en direction longitudinale font entre elles un angle de 30° et deux de ses parois latérales qui s'étendent en direction longitudinale font entre elles un angle de 60°, de preference au moins deux des parois laterales qui s'étendent en direction longitudinale comprenant des fenêtres.

13. Dispositif destiné ä l'émission de rayonnement électromagnétique selon l'une quelconque des revendications 5 à 12, **caractérisé en ce que** le boîtier comprend au moins un connecteur électrique qui est de preference conçu pour être accouplé avec un connecteur d'un autre boîtier et/ou le boîtier est conçu pour être accouplé de façon modulaire et en particulier comprend des moyens de fixation pour l'accouplement modulaire.

14. Dispositif destiné à l'émission de rayonnement électromagnétique selon l'une quelconque des revendications 5 à 13, **caractérisé en ce qu'**à l'intérieur du boîtier du dispositif destiné à l'émission de rayonnement électromagnétique règne une pression réduite par rapport à l'extérieur du boitier et en particulier le boîtier comporte un orifice d'évacuation pouvant être fermé de façon étanche aux fluides.

15. Procédé pour la multiplication ou la culture de matière biologique de preference pour la fabrication de produits pharmaceutiques, en particulier de produits biopharmaceutiques, comprenant
l'introduction de matière biologique ou d'un précurseur de matière biologique dans un photobioréacteur selon l'une quelconque des revendications 1 à 4,
le fait de faire agir un rayonnement électromagnétique qui a ete émis par un dispositif destiné à l'émission de rayonnement électromagnétique selon l'une quelconque des revendications 5 à 14.

16. Procédé pour la preparation de matière biologique et/ou la fabrication de produits pharmaceutiques, notamment de produits biopharmaceutiques, en particulier conformément à un procédé selon la revendication 15, comprenant la fourniture d'un photobioréacteur qui est doté d'au moins un dispositif destiné à l'émission de rayonnement électromagnétique selon l'une quelconque des revendications 5 à 14.
et dans lequel un rayonnement électromagnétique d'une longueur d'onde définie, de preference de 250 nm, est envoye dans le photobioréacteur pendant une durée définie et après cette irradiation une intensité de rayonnement et/ou une longueur d'onde du rayonnement électromagnétique est/sont mesurée(s) sur une large bande ou selectivement ä une longueur d'onde, en particulier à 270 nm, à l'intérieur du bioréacteur et/ou
dans lequel un rayonnement électromagnétique d'une longueur d'onde définie, de preference de 250 nm, est envoyé dans le photobioréacteur pendant une durée définie et après cette irradiation un rayonnement électromagnétique d'une autre longueur d'onde définie, de preference dans une gamme de 620 à 780 nm, est envoye dans le photobioréacteur.

17. Procédé selon la revendication 15 ou 16, dans lequel on utilise des micro-organismes, en particulier egalement des microalgues, levures et bactéries, photo- ou mixotrophes, modifiés par mutagenèse.

18. Procédé selon l'une quelconque des revendications 15 ä 17, dans lequel on soumet à l'autoclavage le photobioréacteur avec son récipient destiné à la reception de milieux fluides qui contiennent de la matière biologique, et le dispositif destiné ä l'émission de rayonnement électromagnétique, alors que le dispositif destiné ä l'émission de rayonnement électromagnétique avec son boîtier est disposé ä l'intérieur du récipient destiné à la reception de matière biologique.
